# EUROPEAN PATENT APPLICATION

(11) **EP 1 229 026 A1**
(43) Date of publication of application: **07.08.2002**
(21) Application number: 00974858.3
(22) Date of filing: 09.11.2000
(51) Int. Cl.: C07D 263/32, C07D 413/12, A61K 31/421, A61K 31/4439, C07C 251/54, A61K 31/195, A61K 31/235, A61P 3/10, A61P 3/06

(54) **ALKOXYIMINOALKANOIC ACID DERIVATIVES**

(30) Priority: 10.11.1999 JP 32031899
(71) Applicant: Takeda Chemical Industries, Ltd., Osaka-shi, Osaka 541-0045 (JP)
(72) Inventor: MOMOSE, Yu, Takarazuka-shi Hyogo 665-0847 (JP); IMOTO, Hiroshi, Kusatsu-shi Shiga 525-0027 (JP); ODAKA, Hiroyuki, Kobe-shi Hyogo 651-1223 (JP); KIMURA, Hiroyuki, Sakai-shi Osaka 590-0975 (JP)
(74) Representative: Lewin, John Harvey
(86) International application number: JP0007878
(87) International publication number: WO0134579

(57) **Abstract**

A compound represented by the formula 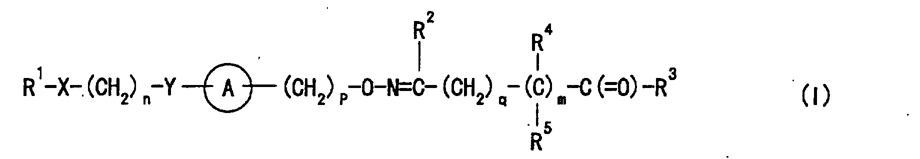 wherein
- R¹: is an optionally substituted hydrocarbon group or an optionally substituted heterocyclic group;
- X: is a bond, an oxygen atom, a sulfur atom, or a group represented by -CO-, -CS-, -CR⁶(OR⁷)- or -NR⁸- (R⁶ and R⁸ are each a hydrogen atom or an optionally substituted hydrocarbon group, and R⁷ is a hydrogen atom or a hydroxy-protecting group);
- n: is an integer of 0 to 3;
- Y: is an oxygen atom, a sulfur atom, or a group represented by -SO-, -SO₂-, -NR⁸-, -CONR⁸- or -NR⁸CO- (R⁸ is a hydrogen atom or an optionally substituted hydrocarbon group);
- ring A: is a heterocycle optionally having 1 to 3 additional substituents or a hydrocarbon ring optionally having 1 to 3 additional substituents;
- p: is an integer of 1 to 8;
- R²: is a hydrogen atom, an optionally substituted hydrocarbon group or an optionally substituted heterocyclic group;
- q: is an integer of 0 to 6;
- m: is 0 or 1;
- R³: is a group represented by -OR⁹ (R⁹ is a hydrogen, atom or an optionally substituted hydrocarbon group) or -NR¹⁰R¹¹ (R¹⁰ and R¹¹ are the same or different and each is a hydrogen atom, an optionally substituted hydrocarbon group, an optionally substituted heterocyclic group, or an optionally substituted acyl group, and R¹⁰ and R¹¹ may be taken together to form a ring);
- R⁴ and R⁵: are the same or different and each is a hydrogen atom or an optionally substituted hydrocarbon group, and R⁴ and R² may be taken together to form a ring, provided that, when ring A is an optionally substituted indole, Y is not an oxygen atom or a sulfur atom; when Y is an oxygen atom, a sulfur atom, -SO-, -SO₂- or -NR⁸- (R⁸ is as defined above), ring A is not a benzene ring optionally having substituents; and when Y is an oxygen atom and ring A is an optionally substituted . 4-pyrone, an optionally substituted 4-pyridone or an optionally substituted pyridine-N-oxide, R² is not a thiazole substituted by an optionally protected amino group or a thiadiazole substituted by an optionally protected amino group,
and a salt thereof are useful as an agent for the prophylaxis or treatment of diabetes and the like.

SEQUENCE LISTING

<110> Takeda Chemical Industries, Ltd.

<120> Alkoxyiminoalkanoic Acid Derivatives

<130> 2667WOOP

<150> JP 11-320318

<151> 1999-11-10

<160> 8

<210> 1

<211> 33

<212> DNA

<213> Artificial Sequence

<220>

<223>

<400> 1

GTGGGTACCG AAATGACCAT GGTTGACACA GAG 33

<210> 2

<211> 33

<212> DNA

<213> Artificial Sequence

<220>

<223>

<400> 2

GGGGTCGACC AGGACTCTCT GCTAGTACAA GTC 33

<210> 3

<211> 33

<212> DNA

<213> Artificial Sequence

<220>

<223>

<400> 3
TTAGAATTCG ACATGGACAC CAAACATTTC CTG     33
<210> 4
<211> 33
<212> DNA
<213> Artificial Sequence
<220>
<223>
<400> 4
CCCCTCGAGC TAAGTCATTT GGTGCGGCGC CTC     33
<210> 5
<211> 36
<212> DNA
<213> Artificial Sequence
<220>
<223>
<400> 5
TCGACAGGGG ACCAGGACAA AGGTCACGTT CGGGAG     36
<210> 6
<211> 36
<212> DNA
<213> Artificial Sequence
<220>
<223>
<400> 6
TCGACTCCCG AACGTGACCT TTGTCCTGGT CCCCTG     36
<210> 7
<211> 28
<212> DNA
<213> Artificial Sequence

<220>

<223>

<400> 7

CCCAGATCTC CCCAGCGTCT TGTCATTG 28

<210> 8

<211> 28

<212> DNA

<213> Artificial Sequence

<220>

<223>

<400> 8

TCACCATGGT CAAGCTTTTA AGCGGGTC 28

## Description

### TECHNICAL FIELD

The present invention relates to novel alkoxyiminoalkanoic acid derivatives having an excellent hypoglycemic action and hypolipidemic action and useful as an agent for the prophylaxis or treatment of diabetes, hyperlipidemia, impaired glucose tolerance, inflammatory diseases, arterial sclerosis and the like.

The present invention also relates to an agent for the prophylaxis or treatment of diabetes, hyperlipidemia or impaired glucose tolerance, which contains an alkoxyiminoalkanoic acid derivative.

The present invention further relates to an agent for regulating a retinoid-related receptor function or an agent for improving insulin resistance, which contains an alkoxyiminoalkanoic acid derivative.

### BACKGROUND ART

As alkoxyiminoalkanoic acid derivatives, there have been reported heretofore, for example, compounds having a leukotriene biosynthesis inhibitory action (e.g., WO96/02507, USP 5399699), and intermediate compounds used for the production of cephalosporin derivatives (e.g., JP-A-4-91088, EP-A 251299, EP-A 435333, EP-A 289002).

However, there is no report on a hypoglycemic action or a hypolipidemic action of these compounds.

Peroxisome proliferator-activated receptor γ (PPARγ) is a member of an intranuclear hormone receptor superfamily represented by steroid hormone receptors and thyroid hormone receptors, whose expression is induced at an extremely early phase of differentiation of adipocytes, and plays an important role for differentiation of adipocytes as a master regulator. PPARγ binds to a ligand to form a dimer with retinoid X receptor (RXR) and the dimer binds to a responsive element of a target gene in the nucleus to directly control (activate) the transcription efficiency. Recently, it has been suggested that 15-deoxy-Δ^{12.14} prostaglandin J₂, which is a metabolite of prostaglandin D₂, is possibly an endogenous ligand of PPARγ, and moreover clarified that certain agents for enhancing insulin sensitivity, typically represented by thiazolidinedione derivatives, have a PPARγ ligand activity and the strength of the activity is in proportion to the hypoglycemic action or adipocyte differentiation promoting action [Cell, vol. 83, p. 803 (1995); The Journal of Biological Chemistry, vol. 270, p. 12953 (1995): Journal of Medicinal Chemistry, vol. 39, p. 655 (1996)]. More recently, it has been elucidated that 1) PPARγ is expressed in the cultured cell derived from human lipbsarcoma and the addition of PPARγ ligand stops its growth [Proceedings of The National Academy of Sciences of The United States of America, vol. 94, p. 237 (1997)], 2) nonsteroidal anti-inflammatory drugs represented by indomethacin and fenoprofen have a PPARγ ligand activity [The Journal of Biological Chemistry, vol. 272, p. 3406 (1997)], 3) PPARγ is highly expressed in activated macrophage, and the addition of its ligand leads to the inhibition of the transcription of the gene involved in inflammation [Nature, vol. 391, p. 79 (1998)], and that 4) PPARγ ligand inhibits production of inflammatory cytokines (TNFα, IL-1β, IL-6) by monocyte [Nature, vol. 391, p. 82 (1998)], and the like.

There is a demand on the development of a novel compound useful as an agent for the prophylaxis or treatment of diabetes, hyperlipidemia, impaired glucose tolerance, inflammatory diseases, arterial sclerosis and the like, which has superior properties as a pharmaceutical agent as evidenced by lower side effects and the like.

### DISCLOSURE OF THE INVENTION

The present invention relates to
1) a compound represented by the formula 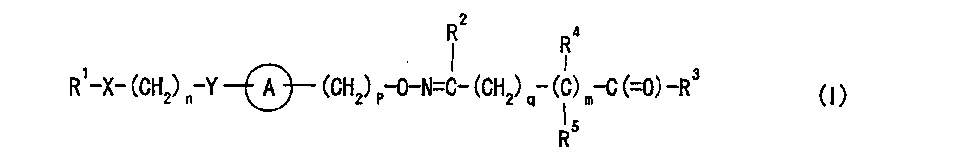 wherein
   - R¹: is an optionally substituted hydrocarbon group or an optionally substituted heterocyclic group;
   - X: is a bond, an oxygen atom, a sulfur atom, or a group represented by -CO-, -CS-, -CR⁶(OR⁷)- or -NR⁸- (R⁶ and R⁸ are each a hydrogen atom or an optionally substituted hydrocarbon group, and R⁷ is a hydrogen atom or a hydroxy-protecting group);
   - n: is an integer of 0 to 3;
   - Y: is an oxygen atom, a sulfur atom, or a group represented by -SO-, -SO₂-, -NR⁸-, -CONR⁸- or -NR⁸CO- (R⁸ is a hydrogen atom or an optionally substituted hydrocarbon group);
   - ring A: is a heterocycle optionally having 1 to 3 additional substituents or a hydrocarbon ring optionally having 1 to 3 additional substituents;
   - p: is an integer of 1 to 8;
   - R²: is a hydrogen atom, an optionally substituted hydrocarbon group or an optionally substituted heterocyclic group;
   - q: is an integer of 0 to 6;
   - m: is 0 or 1;
   - R³: is a group represented by -OR⁹ (R⁹ is a hydrogen atom or an optionally substituted hydrocarbon group) or -NR¹⁰R¹¹ (R¹⁰ and R¹¹ are the same or different and each is a hydrogen atom, an optionally substituted hydrocarbon group, an optionally substituted heterocyclic group, or an optionally substituted acyl group, and R¹⁰ and R¹¹ may be taken together to form a ring);
   - R⁴ and R⁵: are the same or different and each is a hydrogen atom or an optionally substituted hydrocarbon group, and R⁴ and R² may be taken together to form a ring, provided that, when ring A is an optionally substituted indole, Y is not an oxygen atom or a sulfur atom; when Y is an oxygen atom, a sulfur atom, -SO-, -SO₂- or -NR⁸- (R⁸ is as defined above), ring A is not a benzene ring optionally having substituents; and when Y is an oxygen atom and ring A is an optionally substituted 4-pyrone, an optionally substituted 4-pyridone or an optionally substituted pyridine-N-oxide, R² is not a thiazole substituted by an optionally protected amino group or a thiadiazole substituted by an optionally protected amino group,
   or a salt thereof;
2) the compound of the aforementioned 1) wherein R¹ is an optionally substituted heterocyclic group or an optionally substituted cyclic hydrocarbon group;
3) the compound of the aforementioned 1), wherein R¹ is an optionally substituted heterocyclic group;
4) the compound of the aforementioned 1), wherein X is a bond;
5) the compound of the aforementioned 1), wherein n is 1 or 2;
6) the compound of the aforementioned 1), wherein Y is an oxygen atom;
7) the compound of the aforementioned 1), wherein the ring A is a pyridine ring optionally having 1 to 3 additional substituents or a naphthalene ring optionally having 1 to 3 additional substituents;
8) the compound of the aforementioned 1), wherein p is an integer of 1 to 3;
9) the compound of the aforementioned 1), wherein R² is an optionally substituted hydrocarbon group;
10) the compound of the aforementioned 1), wherein q is an integer of 0 to 4;
11) the compound of the aforementioned 1), wherein R³ is a group represented by -OR⁹ (R⁹ is a hydrogen atom or an optionally substituted hydrocarbon group);
12) the compound of the aforementioned 1), which is methyl (E)-4-[6-(5-methyl-2-phenyl-4-oxazolylmethoxy)-2-naphthylmethoxyimino]-4-phenylbutyrate;
   (E)-4-[6-(5-methyl-2-phenyl-4-oxazolylmethoxy)-2-naphthylmethoxyimino]-4-phenylbutyric acid;
   methyl (E)-4-[6-(5-methyl-2-phenyl-4-oxazolylmethoxy).-3-pyridylmethoxyimino]-4-phenylbutyrate; or
   (E)-4-[6-(5-methyl-2-phenyl-4-oxazolylmethoxy)-3-pyridylmethoxyimino]-4-phenylbutyric acid;
13) a prodrug of the compound the aforementioned 1);
14) a pharmaceutical composition comprising a compound represented by the formula (I), a salt thereof or a prodrug thereof;
15) an agent for the prophylaxis or treatment of diabetes, which comprises a compound represented by the formula 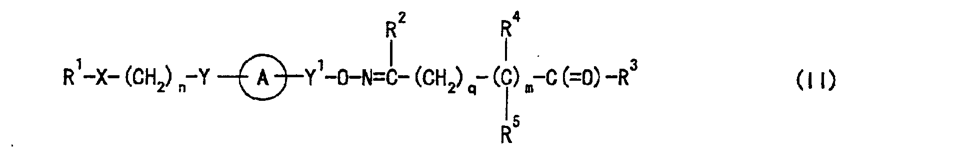 wherein
   - R¹: is an optionally substituted hydrocarbon group or an optionally substituted heterocyclic group;
   - X: is a bond, an oxygen atom, a sulfur atom, or a group represented by -CO-, -CS-, -CR⁶(OR⁷)- or -NR⁸- (R⁶ and R⁸ are each a hydrogen atom or an optionally substituted hydrocarbon group, and R⁷ is a hydrogen atom or a hydroxy-protecting group);
   - n: is an integer of 0 to 3;
   - Y: is an oxygen atom, a sulfur atom, or a group represented by -SO-, -SO₂-, -NR⁸-, -CONR⁸- or -NR⁸CO- (R⁸ is a hydrogen atom or an optionally substituted hydrocarbon group);
   - ring A: is a heterocycle optionally having 1 to 3 additional substituents or a hydrocarbon ring optionally having 1 to 3 additional substituents;
   - Y¹: is an optionally substituted divalent hydrocarbon residue having 1 to 8 carbon atoms in a main chain;
   - R²: is a hydrogen atom, an optionally substituted hydrocarbon group or an optionally substituted heterocyclic group;
   - q: is an integer of 0 to 6;
   - m: is 0 or 1;
   - R³: is a group represented by -OR⁹ (R⁹ is a hydrogen atom or an optionally substituted hydrocarbon group) or -NR¹⁰R¹¹ (R¹⁰ and R¹¹ are the same or different and each is a hydrogen atom, an optionally substituted hydrocarbon group, an optionally substituted heterocyclic group, or an optionally substituted acyl group, and R¹⁰ and R¹¹ may be taken together to form a ring);
   - R⁴ and R⁵: are the same or different and each is a hydrogen atom or an optionally substituted hydrocarbon group, and R⁴ and R² may be taken together to form a ring, provided that, when Y is an oxygen atom, a sulfur atom, -SO-, -SO₂- or -NR⁸- (R⁸ is as defined above), ring A is not a benzene ring optionally having substituents;
   or a salt thereof or a prodrug thereof;
16) an agent for the prophylaxis or treatment of hyperlipidemia, which comprises a compound represented by the formula (II), a salt thereof or a prodrug thereof;
17) an agent for the prophylaxis or treatment of impaired glucose tolerance, which comprises a compound represented by the formula (II), a salt thereof or a prodrug thereof;
18) an agent for regulating a retinoid-related receptor function, which comprises a compound represented by the formula (II), a salt thereof or a prodrug thereof;
19) the agent of the aforementioned 18), which is a ligand for a peroxisome proliferator-activated receptor;
20) the agent of the aforementioned 18), which is a retinoid X receptor ligand;
21) an agent for improving insulin resistance comprising a compound represented by the formula (II), a salt thereof or a prodrug thereof;
22) a method for the prophylaxis or treatment of diabetes, which comprises administering an effective amount of a compound represented by the formula (II), a salt thereof or a prodrug thereof to a mammal;
23) a method for the prophylaxis or treatment of hyperlipidemia, which comprises administering an effective amount of a compound represented by the formula (II), a salt thereof or a prodrug thereof to a mammal;
24) a method for the prophylaxis or treatment of impaired glucose tolerance, which comprises administering an effective amount of a compound represented by the formula (II), a salt thereof or a prodrug thereof to a mammal;
25) a method for regulating a retinoid-related receptor function, which comprises administering an effective amount of a compound represented by the formula (II), a salt thereof or a prodrug thereof to a mammal;
26) use of a compound represented by the formula (II), a salt thereof or a prodrug thereof for the production of an agent for the prophylaxis or treatment of diabetes;
27) use of a compound represented by the formula (II), a salt thereof or a prodrug thereof for the production of an agent for the prophylaxis or treatment of hyperlipidemia;
28) use of a compound represented by the formula (II), a salt thereof or a prodrug thereof for the production of an agent for the prophylaxis or treatment of impaired glucose tolerance;
29) use of a compound represented by the formula (II), a salt thereof or a prodrug thereof for the production of an agent for regulating a retinoid-related receptor function; and the like.

### (1) Definition of R¹

### (1-1) Definition of "hydrocarbon group" in R¹

As the hydrocarbon group of the "optionally substituted hydrocarbon group" represented by R¹ in the formulas (I) and (II), an aliphatic hydrocarbon group, an alicyclic hydrocarbon group, an alicyclic-aliphatic hydrocarbon group, an aromatic aliphatic hydrocarbon group and an aromatic hydrocarbon group are exemplified. These hydrocarbon groups preferably have 1 to 14 carbon atoms.

As the aliphatic hydrocarbon group, an aliphatic hydrocarbon group having 1 to 8 carbon atoms is preferable. As the aliphatic hydrocarbon group, for example, a saturated aliphatic hydrocarbon group having 1 to 8 carbon atoms (e.g., alkyl group and the like), such as methyl, ethyl, propyl, isopropyl, butyl, isobutyl, sec.-butyl, t.-butyl, pentyl, isopentyl, neopentyl, hexyl, isohexyl, heptyl, octyl and the like; and an unsaturated aliphatic hydrocarbon group having 2 to 8 carbon atoms (e.g., alkenyl group having 2 to 8 carbon atoms, alkadienyl group having 4 to 8 carbon atoms, alkenylalkynyl group having 2 to 8 carbon atoms, alkadiynyl group having 4 to 8 carbon atoms and the like), such as ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 2,4-hexadiynyl, 5-hexynyl, 1-heptynyl, 1-octynyl and the like, are mentioned.

As the alicyclic hydrocarbon group, an alicyclic hydrocarbon group having 3 to 7 carbon atoms is preferable. As the alicyclic hydrocarbon group, for example, a saturated alicyclic hydrocarbon group having 3 to 7 carbon atoms (e.g., cycloalkyl group and the like), such as cyclopropyl, cyclobutyl, cyclopentyl, cyclohexyl, cycloheptyl and the like; and an unsaturated alicyclic hydrocarbon group having 5 to 7 carbon atoms (e.g., cycloalkenyl group, cycloalkadienyl group and the like), such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl and the like, are mentioned.

The alicyclic-aliphatic hydrocarbon group is exemplified by those resulting from binding of the aforementioned alicyclic hydrocarbon groups and aliphatic hydrocarbon groups, such as cycloalkyl-alkyl group, cycloalkenyl-alkyl group and the like. Of the alicyclic-aliphatic hydrocarbon groups, one having 4 to 9 carbon atoms is preferable. As the alicyclic-aliphatic hydrocarbon group, for example, cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like are mentioned.

As the aromatic aliphatic hydrocarbon group, an aromatic aliphatic hydrocarbon group having 7 to 13 carbon atoms (e.g., aralkyl group having 7 to 13 carbon atoms, arylalkenyl group having 8 to 13 carbon atoms, and the like) is preferable. As the aromatic aliphatic hydrocarbon group, for example, phenylalkyl having 7 to 9 carbon atoms such as benzyl, phenethyl, 1-phenylethyl, 1-phenylpropyl, 2-phenylpropyl, 3-phenylpropyl and the like; naphthylalkyl having 11 to 13 carbon atoms, such as α-naphthylmethyl, α-naphthylethyl, β-naphthylmethyl, β-naphthylethyl and the like; phenylalkenyl having 8 to 10 carbon atoms such as styryl and the like; and naphthylalkenyl having 12 or 13 carbon atoms such as 2-(2-naphthylvinyl) and the like are mentioned.

As the aromatic hydrocarbon group, an aromatic hydrocarbon group having 6 to 14 carbon atoms (e.g., aryl group and the like) is preferable. As the aromatic hydrocarbon group, for example, phenyl, naphthyl, anthryl, phenanthryl, acenaphthylenyl, biphenylyl and the like are mentioned. Of these, phenyl, 1-naphthyl, 2-naphthyl and the like are preferable.

Of the aforementioned hydrocarbon groups, cyclic. hydrocarbon groups such as alicyclic hydrocarbon group, aromatic hydrocarbon group and the like are preferable. The hydrocarbon group is more preferably an aromatic hydrocarbon group having 6 to 14 carbon atoms, particularly preferably phenyl, naphthyl and the like.

### (1-2) Definition of "heterocyclic group" in R¹

As the heterocyclic group of the "optionally substituted heterocyclic group" represented by R¹ in the formulas (I) and (II), a 5 to 7-membered monocyclic heterocyclic group or a fused heterocyclic group, containing, as a ring-constituting atom besides carbon atom, 1 to 4 heteroatoms selected from oxygen atom, sulfur atom and nitrogen atom is mentioned. As the fused heterocyclic group, for example, a fused ring group of these 5 to 7-membered monocyclic heterocyclic groups with a 6-membered ring containing 1 or 2 nitrogen atoms, benzene ring or a 5-membered ring containing one sulfur atom is mentioned.

Specific examples of the heterocyclic group include aromatic heterocyclic groups, such as 2-furyl, 3-furyl, 2-thienyl, 3-thienyl, 2-pyridyl, 3-pyridyl, 4-pyridyl, 2-pyrimidinyl, 4-pyrimidinyl, 5-pyrimidinyl, 6-pyrimidinyl, 3-pyridazinyl, 4-pyridazinyl, 2-pyrazinyl, 1-pyrrolyl, 2-pyrrolyl, 3-pyrrolyl, 1-imidazolyl, 2-imidazolyl, 4-imidazolyl, 5-imidazolyl, 1-pyrazolyl, 3-pyrazolyl, 4-pyrazolyl, isoxazolyl, isothiazolyl, 2-thiazolyl, 4-thiazolyl, 5-thiazolyl, 2-oxazolyl, 4-oxazolyl, 5-oxazolyl, 1,2,4-oxadizol-3-yl, 1,2,4-oxadiazol-5-yl, 1,3,4-oxadiazol-2-yl, 1,3,4-thiadiazol-2-yl, 1,2,4-triazol-1-yl, 1,2,4-triazol-3-yl, 1,2,3-triazol-1-yl, 1,2,3-triazol-2-yl, 1,2,3-triazol-4-yl, tetrazol-1-yl, tetrazol-5-yl, 2-quinolyl, 3-quinolyl, 4-quinolyl, 2-quinazolyl, 4-quinazolyl, 2-quinoxalyl, 2-benzoxazolyl, 2-benzothiazolyl, benzimidazol-1-yl, benzimidazol-2-yl, indol-1-yl, indol-3-yl, 1H-indazol-3-yl, 1H-pyrrolo[2,3-b]pyrazin-2-yl, 1H-pyrrolo[2,3-b]pyridin-6-yl, 1H-imidazo[4,5-b]pyridin-2-yl, 1H-imidazo[4,5-c]pyridin-2-yl, 1H-imidazo[4,5-b]pyrazin-2-yl, benztriazol-1-yl and the like; and a non-aromatic heterocyclic group, such as 1-pyrrolidinyl, piperidino, morpholino, thiomorpholino, 1-piperazinyl, hexamethylenimin-1-yl, oxazolidin-3-yl, thiazolidin-3-yl, imidazolidin-3-yl, 2-oxoimidazolidin-1-yl, 2,4-dioxoimidazolidin-3-yl, 2,4-dioxooxazolidin-3-yl, 2,4-dioxothiazolidin-3-yl, 1-oxo-phthalazin-2-yl, 2-oxo-2,3-dihydro-4H-1,4-benzothiazin-4-yl and the like; and the like.

The heterocyclic group is preferably an aromatic heterocyclic group, more preferably a nitrogen containing 5-or 6-membered aromatic heterocyclic group optionally fused with a benzene ring, which is preferably furyl, thienyl, pyridyl, pyrimidinyl, pyrazinyl, pyrazolyl, oxazolyl, thiazolyl, triazolyl or oxadiazolyl. Of these, furyl, thienyl, pyridyl, pyrimidinyl, pyrazinyl, pyrazolyl, oxazolyl, thiazolyl, oxadiazolyl, benzoxazolyl, benzothiazolyl, quinolyl and the like are preferable.

### (1-3) Definition of "substituent" in R¹

In the formulas (I) and (II), the hydrocarbon group and the heterocyclic group represented by R¹ each optionally have 1 to 5, preferably 1 to 3, substituents at substitutable positions. As the substituent, for example, "halogen atom", "nitro group", "optionally substituted aliphatic hydrocarbon group", "optionally substituted alicyclic hydrocarbon group", "optionally substituted aromatic hydrocarbon group", "optionally substituted aromatic heterocyclic group", "optionally substituted non-aromatic heterocyclic group", "optionally substituted acyl group", "optionally substituted amino group", "optionally substituted hydroxy group", "optionally substituted thiol group", "optionally esterified or amidated carboxyl group" and the like are mentioned.

As the "halogen atom", fluorine, chlorine, bromine and iodine are exemplified, particularly preferably fluorine and chlorine.

As the aliphatic hydrocarbon group of the "optionally substituted aliphatic hydrocarbon group", a linear or branched aliphatic hydrocarbon group having 1 to 15 carbon atoms is exemplified, such as alkyl group, alkenyl group, alkynyl group and the like.

Preferable examples of the alkyl group include an alkyl group having 1 to 10 carbon atoms, such as methyl, ethyl, propyl, isopropyl, butyl, isobutyl, sec.-butyl, t.-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

Preferable examples of the alkenyl group include an alkenyl group having 2 to 10 carbon atoms, such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

Preferable examples of the alkynyl group include an alkynyl group having 2 to 10 carbon atoms, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the substituent of the "optionally substituted aliphatic hydrocarbon group" include a cycloalkyl group having 3 to 10 carbon atoms, an aryl group having 6 to 14 carbon atoms (e.g., phenyl, naphthyl etc.), an aromatic heterocyclic group (e.g., thienyl, furyl, pyridyl, oxazolyl, thiazolyl etc.), a non-aromatic heterocyclic group (e.g., tetrahydrofuryl, morpholino, thiomorpholino, piperidino, pyrrolidinyl, piperazinyl etc.), an aralkyl group having 7 to 9 carbon atoms, an amino group, an amino group mono- or di-substituted by alkyl group having 1 to 4 carbon atoms or acyl group having 2 to 8 carbon atoms (e.g., alkanoyl group etc.), an amidino group, an acyl group having 2 to 8 carbon atoms (e.g., alkanoyl group etc.), a carbamoyl group, a carbamoyl group mono- or di-substituted by alkyl group having 1 to 4 carbon atoms, a sulfamoyl group, a sulfamoyl group mono- or di-substituted by alkyl group having 1 to 4 carbon atoms, a carboxyl group, an alkoxycarbonyl group having 2 to 8 carbon atoms, hydroxy group, an alkoxy group having 1 to 6 carbon atoms optionally substituted by 1 to 3 halogen atoms (e.g., fluorine, chlorine, bromine, iodine etc.), an alkenyloxy group having 2 to 5 carbon atoms optionally substituted by 1 to 3 halogen atoms (e.g., fluorine, chlorine, bromine, iodine etc.), a cycloalkyloxy group having 3 to 7 carbon atoms, an aralkyloxy group having 7 to 9 carbon atoms, an aryloxy group having 6 to 14 carbon atoms (e.g., phenyloxy, naphthyloxy etc.), a thiol group, an alkylthio group having 1 to 6 carbon atoms optionally substituted by 1 to 3 halogen atoms (e.g., fluorine, chlorine, bromine, iodine etc.), an aralkylthio group having 7 to 9 carbon atoms, an arylthio group having 6 to 14 carbon atoms (e.g., phenylthio, naphthylthio etc.), a sulfo group, a cyano group, an azide group, a nitro group, a nitroso group, a halogen atom (e.g., fluorine, chlorine, bromine, iodine) and the like. The number of substituents is, for example, 1 to 3.

Examples of the alicyclic hydrocarbon group of the "optionally substituted alicyclic hydrocarbon group" include a saturated or unsaturated alicyclic hydrocarbon group having 3 to 12 carbon atoms, such as cycloalkyl group, cycloalkenyl group, cycloalkadienyl group and the like.

Preferable examples of the cycloalkyl group include a cycloalkyl group having 3 to 10 carbon atoms, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Preferable examples of the cycloalkenyl group include a cycloalkenyl group having 3 to 10 carbon atoms, such as 2-cyclopenten-1-yl, 3-cyclopenten-1-yl, 2-cyclohexen-1-yl, 3-cyclohexen-1-yl and the like.

Preferable examples of the cycloalkadienyl group include a cycloalkadienyl group having 4 to 10 carbon atoms, such as 2,4-cyclopentadien-1-yl, 2,4-cyclohexadien-1-yl, 2,5-cyclohexadien-1-yl and the like.

Preferable examples of the aromatic hydrocarbon group of the "optionally substituted aromatic hydrocarbon group" include an aromatic hydrocarbon group having 6 to 14 carbon atoms (e.g., aryl group etc.), such as phenyl, naphthyl, anthryl, phenanthryl, acenaphtylenyl, biphenylyl and the like. Of these, phenyl, 1-naphthyl, 2-naphthyl and the like are preferable.

Preferable examples of the aromatic heterocyclic group of the "optionally substituted aromatic heterocyclic group" include a 5 to 7-membered aromatic monocyclic heterocyclic group containing, as a ring-constituting atom besides carbon atom, 1 to 4 heteroatoms selected from oxygen atom, sulfur atom and nitrogen atom, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl and the like; a bicyclic or tricyclic aromatic fused heterocycle having 3 to 13 carbon atoms, which contains, as a ring-constituting atom besides carbon atom, 1 to 5 heteroatoms selected from oxygen atom, sulfur atom and nitrogen atom, such as benzofuranyl, isobenzofuranyl, benzo[b]thienyl, indolyl, isoindolyl, 1H-indazolyl, benzimidazolyl, benzoxazolyl, benzothiazolyl, 1H-benzotriazolyl, quinolyl, isoquinolyl, cinnolyl, quinazolyl, quinoxalinyl, phthalazinyl, naphthylidinyl, purinyl, pteridinyl, carbazolyl, α-carbonylyl, β-carbonylyl, γ-carbonylyl, acridinyl, phenoxazinyl, phenothiazinyl, phenazinyl, phenoxathiinyl, thianthrenyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like; and the like.

Preferable examples of the non-aromatic heterocyclic group of the "optionally substituted non-aromatic heterocyclic group" include those having 2 to 10 carbon atoms, which contain, as a ring-constituting atom besides carbon atom, 1 to 3 heteroatoms selected from oxygen atom, sulfur atom and nitrogen atom, such as oxiranyl, azetidinyl, oxetanyl, thietanyl, pyrrolidinyl, tetrahydrofuryl, tetrahydropyranyl, morpholinyl, thiomorpholinyl, piperazinyl, pyrrolidinyl, piperidino, morpholino, thiomorpholino and the like.

Examples of the substituent of the aforementioned "optionally substituted alicyclic hydrocarbon group", "optionally substituted aromatic hydrocarbon group", "optionally substituted aromatic heterocyclic group" and "optionally substituted non-aromatic heterocyclic group" include an alkyl group having 1 to 6 carbon atoms optionally substituted by 1 to 3 halogen atoms (e.g., fluorine, chlorine, bromine, iodine and the like), an alkenyl group having 2 to 6 carbon atoms optionally substituted by 1 to 3 halogen atoms (e.g., fluorine, chlorine, bromine, iodine and the like), a cycloalkyl group having 3 to 10 carbon atoms, an aryl group having 6 to 14 carbon atoms (e.g., phenyl, naphthyl and the like), an aromatic heterocyclic group (e.g., thienyl, furyl, pyridyl, oxazolyl, thiazolyl and the like), a non-aromatic heterocyclic group (e.g., tetrahydrofuryl, morpholino, thiomorpholino, piperidino, pyrrolidinyl, piperazinyl and the like), an aralkyl group having 7 to 9 carbon atoms, an amino group, an amino group mono- or di-substituted by alkyl group having 1 to 4 carbon atoms or acyl group having 2 to 8 carbon atoms (e.g., alkanoyl group and the like), an amidino group, an acyl group having 2 to 8 carbon atoms (e.g., alkanoyl group and the like), a carbamoyl group, a carbamoyl group mono- or di-substituted by alkyl group having 1 to 4 carbon atoms, a sulfamoyl group, a sulfamoyl group mono- or di-substituted by alkyl group having 1 to 4 carbon atoms, a carboxyl group, an alkoxycarbonyl group having 2 to 8 carbon atoms, a hydroxy group, an alkoxy group having 1 to 6 carbon atoms optionally substituted by 1 to 3 halogen atoms (e.g., fluorine, chlorine, bromine, iodine and the like), an alkenyloxy group having 2 to 5 carbon atoms optionally substituted by 1 to 3 halogen atoms (e.g., fluorine, chlorine, bromine, iodine and the like), a cycloalkyloxy group having 3 to 7 carbon atoms, an aralkyloxy group having 7 to 9 carbon atoms, an aryloxy group having 6 to 14 carbon atoms (e.g., phenyloxy, naphthyloxy and the like), a thiol group, an alkylthio group having 1 to 6 carbon atoms optionally substituted by 1 to 3 halogen atoms (e.g., fluorine, chlorine, bromine, iodine and the like), an aralkylthio group having 7 to 9 carbon atoms, an arylthio group having 6 to 14 carbon atoms (e.g., phenylthio, naphthylthio and the like), a sulfo group, a cyano group, an azide group, a nitro group, a nitroso group, a halogen atom (e.g., fluorine, chlorine, bromine, iodine), and the like. The number of substituents is, for example, 1 to 3.

The acyl group of the "optionally substituted acyl group" is exemplified by an acyl group having 1 to 13 carbon atoms, specifically formyl, and a group represented by the formula: -COR¹², -SO₂R¹², -SOR¹² or -PO₃R¹²R¹³ wherein R¹² and R¹³ are the same or different and each is a hydrocarbon group or an aromatic heterocyclic group, and the like.

Examples of the hydrocarbon group represented by R¹² or R¹³ include the hydrocarbon groups exemplified for the aforementioned R¹. Of these, an alkyl group having 1 to 10 carbon atoms, a cycloalkyl group having 3 to 10 carbon atoms, an alkenyl group having 2 to 10 carbon atoms, a cycloalkenyl group having 3 to 10 carbon atoms, an aryl group having 6 to 12 carbon atoms are preferable.

Examples of the aromatic heterocyclic group represented by R¹² or R¹³ include the aromatic heterocyclic groups exemplified for the aforementioned R¹. Of these, thienyl, furyl, pyridyl and the like are preferable.

Preferable examples of the acyl group include acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, isonicotinoyl and the like.

The acyl group may have 1 to 3 substituents at substitutable positions. Examples of such substituent include a C₁₋₆ alkyl group optionally substituted by 1 to 3 halogen atoms (e.g., fluorine, chlorine, iodine and the like), a C₁₋₆ alkoxy group optionally substituted by 1 to 3 halogen atoms (e.g., fluorine, chlorine, bromine, iodine and the like), a halogen atom (e.g., fluorine, chlorine, bromine, iodine and the like), a nitro, a hydroxy, an amino and the like.

Examples of the "optionally substituted amino group" include an amino group optionally mono- or di-substituted by alkyl group having 1 to 10 carbon atoms, a cycloalkyl group having 3 to 10 carbon atoms, an alkenyl group having 2 to 10 carbon atoms, a cycloalkenyl group having 3 to 10 carbon atoms, an acyl group having 1 to 13 carbon atoms, an aryl group having 6 to 12 carbon atoms and the like.

As used herein, the acyl group is exemplified by those similar to the aforementioned acyl groups, preferably an alkanoyl group having 2 to 10 carbon atoms and an arylcarbonyl group having 7 to 13 carbon atoms, and the like.

Examples of the substituted amino group include methylamino, dimethylamino, ethylamino, diethylamino, propylamino, dibutylamino, diallylamino, cyclohexylamino, acetylamino, propionylamino, benzoylamino, phenylamino, N-methyl-N-phenylamino and the like.

Examples of the "optionally substituted hydroxy group" include a hydroxy group optionally substituted by an optionally substituted alkyl group having 1 to 10 carbon atoms, an optionally substituted alkenyl group having 2 to 10 carbon atoms, an optionally substituted aralkyl having 7 to 10 carbon atoms, an optionally substituted acyl group having 1 to 13 carbon atoms and an optionally substituted aryl group having 6 to 12 carbon atoms.

Examples of the substituents that these "alkyl group having 1 to 10 carbon atoms", "alkenyl group having 2 to 10 carbon atoms", "aralkyl group having 7 to 10 carbon atoms", "acyl group having 1 to 13 carbon atoms" and "aryl group having 6 to 12 carbon atoms" may have include a halogen atom (e.g., fluorine, chlorine, bromine, iodine and the like), a C₁₋₆ alkoxy group optionally substituted by 1 to 3 halogen atoms (e.g., fluorine, chlorine, bromine, iodine and the like), a hydroxy group, a nitro group, an amino group and the like. The number of substituents is, for example, 1 or 2.

Examples of the substituted hydroxy group include optionally substituted alkoxy group, an optionally substituted alkenyloxy group, an optionally substituted aralkyloxy group, an optionally substituted acyloxy group, an optionally substituted aryloxy group and the like.

Preferable examples of the alkoxy group include an alkoxy group having 1 to 10 carbon atoms, such as methoxy, ethoxy, propoxy, isopropoxy, butoxy, isobutoxy, sec.-butoxy, t.-butoxy, pentyloxy, isopentyloxy, neopentyloxy, hexyloxy, heptyloxy, nonyloxy, cyclobutoxy, cyclopentyloxy, cyclohexyloxy and the like.

Preferable examples of the alkenyloxy group include an alkenyloxy group having 2 to 10 carbon atoms, such as allyloxy, crotyloxy, 2-pentenyloxy, 3-hexenyloxy, 2-cyclopentenylmethoxy, 2-cyclohexenylmethoxy and the like.

Preferable examples of the aralkyloxy group include an aralkyloxy group having 7 to 10 carbon atoms, such as phenyl-C₁₋₄ alkyloxy (e.g., benzyloxy, phenethyloxy etc.) and the like.

Preferable examples of the acyloxy group include an acyloxy group having 2 to 13 carbon atoms, more preferably an alkanoyloxy having 2 to 4 carbon atoms (e.g., acetyloxy, propionyloxy, butyryloxy, isobutyryloxy etc.) and the like.

Preferable examples of the aryloxy group include an aryloxy group having 6 to 14 carbon atoms, such as phenoxy, naphthyloxy and the like.

The above-mentioned alkoxy group, alkenyloxy group, aralkyloxy group, acyloxy group and aryloxy group may have 1 or 2 substituents at substitutable positions. Examples of the substituent include a halogen atom (e.g., fluorine, chlorine, bromine, iodine etc.), a C₁₋₆ alkoxy group optionally substituted by 1 to 3 halogen atoms (e.g., fluorine, chlorine, bromine, iodine etc.), a hydroxy, a nitro, an amino and the like. Examples of the substituted aryloxy group include 4-chlorophenoxy, 2-methoxyphenoxy and the like.

Examples of the optionally substituted thiol group include a thiol group optionally substituted by alkyl having 1 to 10 carbon atoms, a cycloalkyl having 3 to 10 carbon atoms, an aralkyl having 7 to 10 carbon atoms, an acyl having 2 to 13 carbon atoms, an aryl having 6 to 14 carbon atoms, a heteroaryl and the like.

Examples of the substituted thiol group include alkylthio, cycloalkylthio, aralkylthio, acylthio, arylthio, heteroarylthio and the like.

Preferable examples of the alkylthio group include an alkylthio group having 1 to 10 carbon atoms, such as methylthio, ethylthio, propylthio, isopropylthio, butylthio, isobutylthio, sec.-butylthio, t.-butylthio, pentylthio, isopentylthio, neopentylthio, hexylthio, heptylthio, nonylthio and the like.

Preferable examples of the cycloalkylthio group include an cycloalkylthio group having 3 to 10 carbon atoms, such as cyclobutylthio, cyclopentylthio, cyclohexylthio and the like.

Preferable examples of the aralkylthio group include an aralkylthio group having 7 to 10 carbon atoms, such as phenyl-C₁₋₄ alkylthio (e.g., benzylthio, phenethylthio etc.) and the like.

Preferable examples of the acylthio group include an acylthio group having 2 to 13 carbon atoms, more preferably an alkanoylthio group having 2 to 4 carbon atoms (e.g., acetylthio, propionylthio, butyrylthio, isobutyrylthio etc.) and the like.

Preferable examples of the arylthio group include an arylthio group having 6 to 14 carbon atoms, such as phenylthio, naphthylthio and the like.

Preferable examples of the heteroarylthio group include 2-pyridylthio, 3-pyridylthio and the like, as well as 2-imidazolylthio, 1,2,4-triazol-5-ylthio and the like.

In the optionally esterified carboxyl group, examples of the esterified carboxyl group include an alkoxycarbonyl group having 2 to 5 carbon atoms (e.g., methoxycarbonyl, ethoxycarbonyl, propoxycarbonyl, butoxycarbonyl etc.), an aralkyloxycarbonyl group having 8 to 10 carbon atoms (e.g., benzyloxycarbonyl etc.), an aryloxycarbonyl group having 7 to 15 carbon atoms optionally substituted by 1 or 2 alkyl groups having 1 to 3 carbon atoms (e.g., phenoxycarbonyl, p-tolyloxycarbonyl etc.), and the like.

In the optionally amidated carboxyl group, examples of the amidated carboxyl group include a group represented by the formula:

-CON(R¹⁴) (R¹⁵)

wherein R¹⁴ and R¹⁵ are the same or different and each is a hydrogen atom, an optionally substituted hydrocarbon group or an optionally substituted heterocyclic group.

Here, the hydrocarbon group of the "optionally substituted hydrocarbon group" represented by R¹⁴ and R¹⁵ includes, for example, an aliphatic hydrocarbon group, an alicyclic hydrocarbon group and an aromatic hydrocarbon group exemplified for the hydrocarbon group of the "optionally substituted hydrocarbon group" represented by R¹. The heterocyclic group of the "optionally substituted heterocyclic group" represented by R¹⁴ and R¹⁵ includes, for example, a heterocyclic group exemplified for the above-mentioned heterocyclic group of the "optionally substituted heterocyclic group" represented by R¹. The hydrocarbon group and heterocyclic group may have 1 to 3 substituents at substitutable positions, and examples of such substituent include a halogen atom (e.g., fluorine, chlorine, bromine, iodine etc.), a C₁₋₆ alkyl group optionally substituted by 1 to 3 halogen atoms (e.g., fluorine, chlorine, bromine, iodine and the like), a C₁₋₆ alkoxy group optionally substituted by 1 to 3 halogen atoms (e.g., fluorine, chlorine, bromine, iodine and the like), a nitro, a hydroxy, an amino and the like.

In the formulas (I) and (II), the substituent of the hydrocarbon group and heterocyclic group represented by R¹ preferably includes
1) an alkyl group having 1 to 10 (preferably 1 to 4) carbon atoms and optionally having 1 to 3 substituents selected from alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino;
2) a cycloalkyl group having 3 to 10 (preferably 3 to 7) carbon atoms and optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino;
3) an aromatic heterocyclic group (preferably furyl, thienyl and the like) optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino;
4) an aryl group having 6 to 14 carbon atoms (preferably phenyl, naphthyl and the like) and optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino; and.the like. The number of substituents is, for example, 1 to 3, preferably 1 or 2.

The substituent is more preferably an alkyl group having 1 to 4 carbon atoms, furyl, thienyl, phenyl, naphthyl and the like.

### (1-4) Preferable examples of R¹

In the formulas (I) and (II), R¹ is preferably an optionally substituted heterocyclic group or an optionally substituted cyclic hydrocarbon group. R¹ is more preferably an optionally substituted heterocyclic group. As used herein, the heterocyclic group is preferably a nitrogen containing 5-or 6-membered aromatic heterocyclic group optionally fused with a benzene ring (preferably furyl, thienyl, pyridyl, pyrimidinyl, pyrazinyl, oxazolyl, thiazolyl, triazolyl, oxadiazolyl, pyrazolyl). Of these, furyl, thienyl, pyridyl, pyrimidinyl, pyrazinyl, oxazolyl, thiazolyl, oxadiazolyl, benzoxazolyl, benzothiazolyl, quinolyl, pyrazolyl and the like are preferable.

Preferable examples of the substituent that the aforementioned heterocyclic group or cyclic hydrocarbon group may have include 1) furyl, thienyl, phenyl or naphthyl each optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino; 2) an alkyl group having 1 to 4 carbon atoms or a cycloalkyl having 3 to 7 carbon atoms, each of which may have 1 to 3 substituents selected from alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino; and the like. The number of substituents is, for example, 1 or 2.

R¹ is particularly preferably pyridyl, oxazolyl, thiazolyl, triazolyl or pyrazolyl, each optionally having 1 or 2 substituents selected from alkyl group having 1 to 3 carbon atoms, cycloalkyl group having 3 to 7 carbon atoms, furyl, thienyl, pyridyl, phenyl and naphthyl.

### (2) Definition of X

In the formulas (I) and (II), X is a bond, an oxygen atom, a sulfur atom, a group represented by -CO-, -CS-, -CR⁶(OR⁷)- or -NR⁸- (R⁶ and R⁸ are each a hydrogen atom or an optionally substituted hydrocarbon group, R⁷ is a hydrogen atom or a hydroxy-protecting group). X is preferably a bond, -CR⁶(OR⁷)- or -NR⁸- (the symbols are as defined above), more preferably a bond or -NR⁸- (the symbol is as defined above). X is particularly preferably a bond.

As used herein, the "optionally substituted hydrocarbon group" represented by R⁶ and R⁸ includes, for example, the "optionally substituted hydrocarbon group" exemplified for the aforementioned R¹. The "optionally substituted hydrocarbon group" is preferably an optionally substituted alkyl group having 1 to 4 carbon atoms, such as methyl, ethyl, propyl, isopropyl, butyl, isobutyl, sec.-butyl, t.-butyl and the like. The alkyl group may have 1 to 3 substituents at substitutable positions. Examples of the substituent include a halogen atom (e.g., fluorine, chlorine, bromine, iodine), an alkoxy group having 1 to 4 carbon atoms (e.g., methoxy, ethoxy, propoxy, isopropoxy, butoxy, isobutoxy, sec.-butoxy, t.-butoxy etc.), a hydroxy, a nitro, an amino, an acyl group having 1 to 4 carbon atoms (e.g., alkanoyl group having 1 to 4 carbon atoms such as formyl, acetyl, propionyl etc.), and the like.

The hydroxy-protecting group represented by R⁷ is exemplified by C₁₋₆ alkyl (e.g., methyl, ethyl, propyl, isopropyl, butyl, tert-butyl and the like), phenyl, trityl, C₇₋₁₀ aralkyl (e.g., benzyl and the like), formyl, C₁₋₆ alkyl-carbonyl (e.g., acetyl, propionyl and the like), benzoyl, C₇₋₁₀ aralkyl-carbonyl (e.g., benzylcarbonyl and the like), 2-tetrahydropyranyl, 2-tetrahydrofuranyl, silyl (e.g., trimethylsilyl, triethylsilyl, dimethylphenylsilyl, tert-butyldimethylsilyl, tert-butyldiethylsilyl and the like), C₂₋₆ alkenyl (e.g., 1-allyl and the like) and the like. These groups are optionally substituted by 1 to 3 halogen atoms (e.g., fluorine, chlorine, bromine, iodine and the like), C₁₋₆ alkyl (e.g., methyl, ethyl, propyl and the like), C₁₋₆ alkoxy (e.g., methoxy, ethoxy, propoxy and the like), nitro and the like.

### (3) Definitions of n and Y

In the formulas (I) and (II), n is an integer of 0 to 3, preferably an integer of 1 to 3, more preferably 1 or 2.

In the formulas (I) and (II), Y is an oxygen atom, a sulfur atom, a group represented by -SO-, -SO₂-, -NR⁸-, -CONR⁸- or -NR⁸CO- (R⁸ is a hydrogen atom or an optionally substituted hydrocarbon group), with preference given to an oxygen atom, a sulfur atom or -NR⁸- (the symbol is as defined above).

Y is particularly preferably an oxygen atom.

### (4) Definition of ring A

In the formulas (I) and (II), the "heterocycle" of the "heterocycle optionally having 1 to 3 additional substituents" represented by ring A is exemplified by a 5- or 6-membered aromatic heterocycle, a 5- or 6-membered non-aromatic heterocycle, a fused aromatic heterocycle and the like.

Examples of the "5- or 6-membered aromatic heterocycle" include a 5- or 6-membered aromatic heterocycle having, besides carbon atom, 1 to 3 heteroatoms selected from nitrogen atom, sulfur atom and oxygen atom, and the like. Specific examples thereof include thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, furazan and the like.

As the "5- or 6-membered non-aromatic heterocycle", for example, a 5- or 6-membered non-aromatic heterocycle containing, besides carbon atom, 1 to 3 heteroatoms selected from nitrogen atom, sulfur atom and oxygen atom, and the like can be mentioned. Specific examples thereof include pyrrolidine, imidazolidine, pyrazolidine, piperidine, piperazine, morpholine, thiomorpholine and the like.

As the "fused aromatic heterocycle", for example, a 9-to 14-membered (preferably 9- or 10-membered) fused aromatic heterocycle containing, besides carbon atom, 1 to 4 heteroatoms selected from nitrogen atom, sulfur atom and oxygen atom, and the like can be mentioned. Specific examples thereof include benzofuran, benzothiophene, benzimidazole, benzoxazole, benzothiazole, benzisothiazole, naphtho[2,3-b]thiophene, isoquinoline, quinoline, indole, quinoxaline, phenanthridine, phenothiazine, phenoxazine, phthalazine, naphthyridine, quinazolin, cinnoline, carbazole, β-carboline, acridine, phenazine, phthalimide and the like.

As the "hydrocarbon ring" of the "hydrocarbon ring optionally having 1 to 3 additional substituents" represented by ring A, for example, a benzene ring, a cycloalkane having 5 to 11 carbon atoms, a fused aromatic hydrocarbon ring having 9 to 14 carbon atoms and the like can be mentioned.

As the "cycloalkane having 5 to 11 carbon atoms", for example, cyclopentane, cyclohexane, cycloheptane, cyclooctane, cyclononane, cyclodecane, cycloundecane and the like can be mentioned.

As the "fused aromatic hydrocarbon ring having 9 to 14 carbon atoms", for example, naphthalene, indene, fluorene, anthracene and the like can be mentioned.

The ring A is preferably "a 5- or 6-membered aromatic heterocycle (preferably pyridine, isoxazole and the like) optionally having 1 to 3 additional substituents or a fused aromatic hydrocarbon ring having 9 to 14 carbon atoms (preferably naphtharene and the like) optionally having 1 to 3 additional substituents."

The "substituent" of the "heterocycle optionally having 1 to 3 additional substituents or a hydrocarbon ring optionally having 1 to 3 additional substituents" represented by ring A includes, for example, an optionally substituted aliphatic hydrocarbon group (preferably alkyl group), an optionally substituted hydroxy group, a halogen atom, an optionally substituted acyl group, a nitro group, an optionally substituted amino group and the like. These substituents are those exemplified for the substituent of R¹. The substituent is preferably an alkyl group having 1 to 4 carbon atoms, an alkoxy group having 1 to 4 carbon atoms or a halogen atom.

The ring A is preferably a "pyridine ring optionally having 1 to 3 additional substituents or a naphthalene ring optionally having 1 to 3 additional substituents", more preferably a pyridine ring or a naphthalene ring, each of which may have 1 to 3 additional substituents selected from alkyl group having 1 to 4 carbon atoms, alkoxy group having 1 to 4 carbon atoms and halogen atom.

### (5) Definition of p

In the formulas (I) and (II), p is an integer of 1 to 8, preferably an integer of 1 to 3.

### (6) Definition of R²

In the formulas (I) and (II), the "optionally substituted hydrocarbon group" and "optionally substituted heterocyclic group" represented by R² respectively include, for example, an "optionally substituted hydrocarbon group" and an "optionally substituted heterocyclic group" exemplified for R¹.

R² is preferably an optionally substituted hydrocarbon group. The hydrocarbon group is preferably an aromatic hydrocarbon group having 6 to 14 carbon atoms (e.g., phenyl, naphthyl and the like). The substituent that the hydrocarbon group may have is preferably a halogen atom (e.g., fluorine, chlorine, bromine, iodine), an alkyl group having 1 to 4 carbon atoms optionally substituted by 1 to 3 halogen atoms (e.g., fluorine, chlorine, bromine, iodine), an alkoxy group having 1 to 4 carbon atoms optionally substituted by 1 to 3 halogen atoms (e.g., fluorine, chlorine, bromine, iodine), an aryloxy group having 6 to 14 carbon atoms, an aromatic heterocyclic group (e.g., furyl, thienyl) and the like. The number of substituents is, for example, 1 to 3.

R² is more preferably an aromatic hydrocarbon group having 6 to 14 carbon atoms (e.g., phenyl, naphthyl and the like), which may have 1 to 3 substituents selected from halogen atom (e.g., fluorine, chlorine, bromine, iodine), alkyl group having 1 to 4 carbon atoms optionally substituted by 1 to 3 halogen atoms (e.g., fluorine, chlorine, bromine, iodine), alkoxy group having 1 to 4 carbon atoms optionally substituted by 1 to 3 halogen atoms (e.g., fluorine, chlorine, bromine, iodine), aryloxy group having 6 to 14 carbon atoms and aromatic heterocyclic group (e.g., furyl, thienyl). R² is particularly preferably a phenyl and the like.

### (7) Definition of q and m

In the formulas (I) and (II), q is an integer of 0 to 6, preferably 0 to 4, and m is 0 or 1.

### (8) Definition of R³

In the formulas (I) and (II), R³ is a group represented by -OR⁹ (R⁹ is a hydrogen atom or an optionally substituted hydrocarbon group) or -NR¹⁰R¹¹ (R¹⁰ and R¹¹ are the same or different and each is a hydrogen atom, an optionally substituted hydrocarbon group, an optionally substituted heterocyclic group or an optionally substituted acyl group, and R⁹ and R¹⁰ may be taken together to form a ring).

As the "optionally substituted hydrocarbon group" represented by R⁹, the "optionally substituted hydrocarbon group" exemplified for R¹ is mentioned.

The "optionally substituted hydrocarbon group" is preferably an "alkyl group having 1 to 4 carbon atoms", an "aryl group having 6 to 10 carbon atoms, which may have 1 to 3 substituents selected from alkyl group having 1 to 4 carbon atoms and halogen atom (e.g., fluorine, chlorine, bromine, iodine)" and the like. As used herein, examples of the "alkyl group having 1 to 4 carbon atoms" include methyl, ethyl, propyl, butyl, isobutyl, sec-butyl, t-butyl, particularly preferably methyl and ethyl, and the like. As the "halogen atom", chlorine is preferable. As the "aryl group having 6 to 10 carbon atoms", phenyl and naphthyl can be mentioned, with preference given to phenyl.

The "optionally substituted hydrocarbon group" and the "optionally substituted heterocyclic group" represented by R¹⁰ and R¹¹ respectively include the "optionally substituted hydrocarbon group" and "optionally substituted heterocyclic group" exemplified for R¹.

As the "optionally substituted acyl group" represented by R¹⁰ and R¹¹, the "optionally substituted acyl group" exemplified for the substituent of R¹ can be mentioned.

The ring formed by R¹⁰ and R¹¹ in combination is, for example, a 5- to 7-membered cyclic amino group, which is preferably 1-pyrrolidinyl, 1-piperidinyl, 1-hexamethyleniminyl, 4-morpholino, 4-thiomorpholino and the like.

R¹⁰ and R¹¹ are each preferably a hydrogen atom or an alkyl group having 1 to 4 carbon atoms (e.g., methyl, ethyl).

R³ is preferably a group represented by -OR⁹ (the symbol is as defined above) and R⁹ is preferably a hydrogen atom or an alkyl group having 1 to 4 carbon atoms (e.g., methyl, ethyl).

### (9) Definitions of R⁴ and R⁵

In the formulas (I) and (II), R⁴ and R⁵ are the same or different and each is a hydrogen atom or an optionally substituted hydrocarbon group, and R⁴ and R² may be taken together to form a ring.

The "optionally substituted hydrocarbon group" represented by R⁴ or R⁵ includes the aforementioned "optionally substituted hydrocarbon group" exemplified for R¹, preferably the aforementioned "optionally substituted alkyl group having 1 to 4 carbon atoms" exemplified for R⁶ and R⁸.

Examples of the ring formed by R⁴ and R² in combination include a cycloalkane having 5 to 11 carbon atoms and a cycloalkene having 5 to 11 carbon atoms and the like. Of these, cyclopentane, cyclopentene, cyclohexane, cyclohexene, cycloheptane, cycloheptene, cyclooctane, cyclooctene, cyclononane, cyclononene, cyclodecane, cyclodecene, cycloundecane, cycloundecene and the like are preferable.

R⁴ and R⁵ are the same or different and each is preferably a hydrogen atom or an alkyl group having 1 to 4 carbon atoms.

### (10) Definition of Y¹

In the formula (II), as the "divalent hydrocarbon residue having 1 to 8 carbon atoms in a main chain" of the "optionally substituted divalent hydrocarbon residue having 1 to 8 carbon atoms in a main chain" represented by Y¹, for example, a "divalent acyclic hydrocarbon residue" and a "divalent cyclic hydrocarbon residue" are mentioned.

As the "divalent acyclic hydrocarbon residue", an alkylene having 1 to 8 carbon atoms, an alkenylene having 2 to 8 carbon atoms, an alkynylene.having 2 to 8 carbon atoms and the like can be mentioned.

As the "divalent cyclic hydrocarbon residue", a divalent group obtained by removing optional two hydrogen atoms from a cycloalkane having 5 to 8 carbon atoms, a cycloalkene having 5 to 8 carbon atoms or an aromatic hydrocarbon having 6 to 14 carbon atoms (e.g., benzene, naphtharene, indene, anthracene and the like), and the like can be mentioned. Examples thereof include 1,2-cyclopentylene, 1,3-cyclopentylene, 1,2-cyclohexylene, 1,3-cyclohxylene, 1,4-cyclohxylene, 1,2-cycloheptylene, 1,3-cycloheptylene, 1,4-cycloheptylene, 3-cyclohexen-1,4-ylene, 3-cyclohexen-1,2-ylene, 2,5-cyclohexadien-1,4-ylene, 1,2-phenylene, 1,3-phenylene, 1,4-phenylene, 1,4-naphthylene, 1,6-naphthylene, 2,6-naphthylene, 2,7-naphthylene, 1,5-indenylene, 2,5-indenylene and the like.

The "number of carbon(s) in the main chain" is counted such that the number of atoms present between the ring A and "O-N" is minimum. For example, when the divalent hydrocarbon residue is 1,2-cyclopentylene, 1,3-cyclopentylene, 1,4-phenylene, 2,5-naphthylene or 2,6-naphthylene, the number of atoms in the main chain is counted to be 2, 3, 4, 5 or 6, respectively.

The "divalent hydrocarbon residue having 1 to 8 carbon atoms in a main chain" is preferably
(1) C₁₋₈ alkylene (e.g., -CH₂-, -(CH₂)₂-, -(CH₂)₃-, -(CH₂)₄-, - (CH₂)₅-, -(CH₂)₆-, -(CH₂)₇-, -(CH₂)₈-, -CH(CH₃)-, -C(CH₃)₂-, -(CH(CH₃))₂-, -(CH₂)₂C(CH₃)₂-, -(CH₂)₃C(CH₃)₂- and the like) ;
(2) C₂₋₈ alkenylene (e.g., -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- and the like); or
(3) C₂₋₈ alkynylene (e.g., -C≡C-, -CH₂-C≡C-, -CH₂-C≡C-CH₂-CH₂- and the like), more preferably -(CH₂)ₚ- (the symbol is as defined above).

The "substituent" of the "optionally substituted divalent hydrocarbon residue having 1 to 8 carbon atoms in a main chain" includes, for example, a halogen atom (e.g., fluorine, chlorine, bromine, iodine and the like), a C₁₋₆ alkoxy group optionally substituted by 1 to 3 halogen atoms (e.g., fluorine, chlorine, bromine, iodine and the like), a hydroxy, a nitro, an amino and the like. The number of substituents is, for example, 1 to 3.

Y¹ is preferably -(CH₂)ₚ- (the symbol is as defined above).

### (11) (E) form and/or (Z) form compound(s)

The compound represented by the formula (I) or (II) includes an (E) form and a (Z) form at an imino bond. The compound includes these (E) form and (Z) form singly and a mixture thereof.

In the formula (I), when ring A is an optionally substituted indole, Y is not an oxygen atom or a sulfur atom; when Y is an oxygen atom, sulfur atom, -SO-, -SO₂- or -NR⁸- (the symbol is as defined above), ring A is not a benzene ring optionally having substituents; and when Y is an oxygen atom and ring A is an optionally substituted 4-pyrone, an optionally substituted 4-pyridone (including 4-hydroxypyridine, which is a tautomer)) or an optionally substituted pyridine-N-oxide, R² is not a thiazole or a thiadiazole each substituted by an optionally protected amino group.

In the formula (II), when Y is an oxygen atom, a sulfur atom, -SO-, -SO₂- or -NR⁸- (R⁸ is as defined above), ring A is not a benzene optionally having substituents.

### (12) Preferable compound

Examples of the preferable compound represented by the formula (I) include the following compounds and the like.

A compound wherein
R¹ is a nitrogen-containing 5- or 6-membered aromatic heterocyclic group optionally fused with a benzene ring (preferably furyl, thienyl, pyridyl, pyrimidinyl, pyrazinyl, oxazolyl, thiazolyl, triazolyl, oxadiazolyl, pyrazolyl), which may have 1 or 2 substituents selected from 1) furyl, thienyl; phenyl or naphthyl, each optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino; and 2) alkyl group having 1 to 4 carbon atoms or cycloalkyl having 3 to 7 carbon atoms, each of which may have 1 to 3 substituents selected from alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino;
X is a bond or -NR⁸- (R⁸ is a hydrogen atom or an alkyl group having 1 to 4 carbon atoms);
n is 1 or 2;
Y is an oxygen atom, a sulfur atom or -NR⁸- (R⁸ is a hydrogen atom or an alkyl group having 1 to 4 carbon atoms); ring A is a 5- or 6-membered aromatic heterocycle (preferably pyridine, isoxazole and the like) or a fused aromatic hydrocarbon ring having 9 to 14 carbon atoms (preferably naphtharene and the like), each of which may have 1 to 3 additional substituents selected from alkyl group having 1 to 4 carbon atoms, alkoxy group having 1 to 4 carbon atoms and halogen atom;
p is an integer of 1 to 3;
R² is an aromatic hydrocarbon group having 6 to 14 carbon atoms (e.g., phenyl, naphthyl and the like), which may have 1 to 3 substituents selected from halogen atom (e.g., fluorine, chlorine, bromine, iodine), alkyl group having 1 to 4 carbon atoms optionally substituted by 1 to 3 halogen atoms (e.g., fluorine, chlorine, bromine, iodine), alkoxy group having 1 to 4 carbon atoms optionally substituted by 1 to 3 halogen atoms (e.g., fluorine, chlorine, bromine, iodine), aryloxy group having 6 to 14 carbon atoms and aromatic heterocyclic group (e.g., furyl, thienyl);
q is an integer of 0 to 4;
m is 0 or 1;
R⁴ and R⁵ are the same or different and each is a hydrogen atom or an alkyl group having 1 to 4 carbon atoms; and
R³ is -OR⁹ (R⁹ is a hydrogen atom, an "alkyl group having 1 to 4 carbon atoms" or an "aryl group having 6 to 10 carbon atoms, which may have 1 to 3 substituents selected from alkyl group having 1 to 4 carbon atoms and halogen atom (e.g., fluorine, chlorine, bromine, iodine)") or NR⁹R¹⁰ (R⁹ and R¹⁰ are the same or different and each is a hydrogen atom or an alkyl group having 1 to 4 carbon atoms).

Examples of the preferable compound of the formula (II) include the following compound and the like.

A compound wherein
R¹ is a nitrogen-containing 5- or 6-membered aromatic heterocyclic group optionally fused with a benzene ring (preferably furyl, thienyl, pyridyl, pyrimidinyl, pyrazinyl, oxazolyl, thiazolyl, triazolyl, oxadiazolyl, pyrazolyl), which may have 1 or 2 substituents selected from 1) furyl, thienyl, phenyl or naphthyl, each optionally having 1 to 3 substituents selected from alkyl group having 1 to 6 carbon atoms optionally substituted by 1 to 3 halogen atoms (e.g., fluorine, chlorine, bromine, iodine and the like), alkoxy group having 1 to 6 carbon atoms, which is optionally substituted by 1 to 3 halogen atoms (e.g., fluorine, chlorine, bromine, iodine and the like), halogen atom (e.g., fluorine, chlorine, bromine, iodine and the like), nitro, hydroxy and amino; and 2) alkyl group having 1 to 4 carbon atoms or cycloalkyl having 3 to 7 carbon atoms, each of which may have 1 to 3 substituents selected from alkoxy group having 1 to 6 carbon atoms optionally substituted by 1 to 3 halogen atoms (e.g., fluorine, chlorine, bromine, iodine and the like), halogen atom (e.g., fluorine, chlorine, bromine, iodine and the like), nitro, hydroxy and amino;
X is a bond or -NR⁸- (R⁸ is a hydrogen atom or an alkyl group having 1 to 4 carbon atoms);
n is 1 or 2;
Y is an oxygen atom, a sulfur atom or -NR⁸- (R⁸ is a hydrogen atom or an alkyl group having 1 to 4 carbon atoms); ring A is 5- or 6-membered aromatic heterocycle (preferably pyridine, isoxazole and the like) or a fused aromatic hydrocarbon ring having 9 to 14 carbon atoms (preferably naphtharene and the like), each of which may have 1 to 3 additional substituents selected from alkyl group having 1 to 4 carbon atoms, alkoxy group having 1 to 4 carbon atoms and halogen atom;
Y¹ is a C₁₋₈ alkylene, a C₂₋₈ alkenylene or a C₂₋₈ alkynylene, each optionally having 1 to 3 substituents selected from halogen atom (e.g., fluorine, chlorine, bromine, iodine and the like), C₁₋₆ alkoxy group optionally substituted by 1 to 3 halogen atoms (e.g., fluorine, chlorine, bromine, iodine and the like), hydroxy, nitro and amino [Y¹ is preferably -(CH₂)ₚ- (p is an integer of 1 to 3);
R² is an aromatic hydrocarbon group having 6 to 14 carbon atoms (e.g., phenyl, naphthyl and the like), which may have 1 to 3 substituents selected from halogen atom (e.g., fluorine, chlorine, bromine, iodine), alkyl group having 1 to 4 carbon atoms optionally substituted by 1 to 3 halogen atoms (e.g., fluorine, chlorine, bromine, iodine), alkoxy group having 1 to 4 carbon atoms optionally substituted by 1 to 3 halogen atoms (e.g., fluorine, chlorine, bromine, iodine), aryloxy group having 6 to 14 carbon atoms and aromatic heterocyclic group (e.g., furyl, thienyl);
q is an integer of 0 to 4;
m is 0 or 1;
R⁴ and R⁵ are the same or different and each is a hydrogen atom or an alkyl group having 1 to 4 carbon atoms; and
R³ is -OR⁹ (R⁹ is a hydrogen atom, an "alkyl group having 1 to 4 carbon atoms" or an "aryl group having 6 to 10 carbon atoms, which may have 1 to 3 substituents selected from alkyl group having 1 to 4 carbon atoms and halogen atom (e.g., fluorine, chlorine, bromine, iodine)") or NR⁹R¹⁰ (R⁹ and R¹⁰ are the same or different and each is a hydrogen atom or an alkyl group having 1 to 4 carbon atoms).

### (13) Salt

The salt of the compound represented by the formula (I) or (II) (hereinafter sometimes to be abbreviated as compound (I) or (II) respectively) is preferably a pharmacologically acceptable salt, such as salt with inorganic base, salt with organic base, salt with inorganic acid, salt with organic acid, salt with basic or acidic amino acid and the like.

Preferable examples of the salt with inorganic base include alkali metal salts such as sodium salt, potassium salt and the like; alkaline earth metal salts such as calcium salt, magnesium salt and the like; aluminum salt; ammonium salt; and the like.

Preferable examples of the salt with organic base include salts with trimethylamine, triethylamine, pyridine, picoline, ethanolamine, diethanolamine, triethanolamine, dicyclohexylamine, N,N-dibenzylethylenediamine and the like.

Preferable examples of the salt with inorganic acid include salts with hydrochloric acid, hydrobromic acid, nitric acid, sulfuric acid, phosphoric acid and the like.

Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysin, ornithine and the like.

Preferable examples of the salt with acidic amino acid include salts with aspartic acid, glutamic acid and the like.

Of the above-mentioned salts, sodium salt, potassium salt, hydrochloride and the like are preferable.

### (14) Prodrug and the like

A prodrug of compound (II) is a compound to be converted to compound (II) by the reaction caused by enzymes, gastric acid and the like under the physiological conditions in the body. That is, it refers to a compound to be converted to compound (II) by enzymatic oxidation, reduction, hydrolysis and the like, and a compound to be converted to compound (II) by hydrolysis and the like caused by gastric acid and the like. A prodrug of compound (II) is exemplified by a compound wherein an amino group of compound (II) is acylated, alkylated, phosphorylated (e.g., compound where amino group of compound (II) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated and the like); compound wherein a hydroxy group of compound (II) is acylated, alkylated, phosphorylated, borated (e.g., compound where hydroxy group of compound (II) is acetylated, palmitoylated, propanoylated, pivaloylated, succinilated, fumarilated, alanilated, dimethylaminomethylcarbonylated and the like); compound wherein a carboxyl group of compound (II) is esterified or amidated (e.g., compound where carboxyl group of compound (II) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methylamidated and the like) and the like. These compounds can be produced from compound (II) by a method known *per se.*

Alternatively, the prodrug of compound (II) may be a compound to be converted to compound (II) under physiological conditions as described in Development of pharmaceutical products, vol. 7, Molecule Design, 163-198, Hirokawa Shoten (1990).

The prodrug of compound (I) is similar to those exemplified for the prodrug of compound (II).

The compounds (I) and (II) may be labeled with isotope (e.g., ³H, ¹⁴C, ³⁵S, ¹²⁵I and the like) and the like.

The compounds (I) and (II) may be an anhydride or a hydrate.

### (15) Formulation

The compounds (I), (II) and salts thereof (hereinafter sometimes to be simply referred to as the compound of the present invention) have low toxicity and can be used as an agent for the prophylaxis or treatment of various diseases to be mentioned later in mammals (e.g., human, mouse, rat, rabbit, dog, cat, cattle, horse, swine, simian and the like) as it is or by admixing with a pharmacologically acceptable carrier and the like to give a pharmaceutical composition.

Here, various organic or inorganic carriers conventionally used as materials for pharmaceutical preparations are used as a pharmacologically acceptable carrier, which is admixed as excipient, lubricant, binder, disintegrant for solid preparations; solvent, dissolution aids, suspending agent, isotonicity agent, buffer, soothing agent for liquid preparations and the like. Where necessary, additives for pharmaceutical preparations such as antiseptic, antioxidant, coloring agent, sweetening agent and the like can be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, acacia, dextrin, pullulan, light anhydrous silicic acid, synthetic aluminum silicate, magnesium aluminate metasilicate and the like.

Preferable examples of the lubricant include magnesium stearate, calcium stearate, talc, colloidal silica and the like.

Preferable examples of the binder include pregelatinized starch, saccharose, gelatin, acacia, methylcellulose, carboxymethylcellulose, sodium carboxymethylcellulose, crystalline cellulose, sucrose, D-mannitol, trehalose, dextrin, pullulan, hydroxypropyl cellulose, hydroxypropylmethylcellulose, polyvinylpyrrolidone and the like.

Preferable examples of the disintegrant include lactose, sucrose, starch, carboxymethylcellulose, calcium carboxymethylcellulose, sodium crosscarmellose, sodium carboxymethyl starch, light anhydrous silicic acid, low-substituted hydroxypropylcellulose and the like.

Preferable examples of the solvent include water for injection, physiological brine, Ringer's solution, alcohol, propylene glycol, polyethylene glycol, sesame oil, corn oil, olive oil, cotton seed oil and the like.

Preferable examples of the dissolution aids include polyethylene glycol, propylene glycol, D-mannitol, trehalose, benzyl benzoate, ethanol, Tris aminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, monostearic glyceride and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil and the like.

Preferable examples of the isotonicity agent include sodium chloride, glycerin, D-mannitol, D-sorbitol, glucose and the like.

Preferable examples of the buffer include phosphate buffer, acetate buffer, carbonate buffer, citrate buffer, and the like.

Preferable examples of the soothing agent include benzyl alcohol and the like.

Preferable examples of the antiseptic include paraoxybenzoic acid esters, chlorobutanol, benzyl alcohol, phenethyl alcohol, dehydroacetic acid, sorbic acid and the like.

Preferable examples of the antioxidant include sulfite, ascprbate and the like.

Preferable examples of the coloring agent include water-soluble edible tar pigment (e.g., foodcolors such as Food Color Red Nos. 2 and 3, Food Color Yellow Nos. 4 and 5, Food Color Blue Nos. 1 and 2 and the like, water insoluble lake pigment (e.g., aluminum salt of the aforementioned water-soluble edible tar pigment and the like), natural pigments (e.g., β-carotene, chlorophyll, red ferric oxide etc.) and the like.

Preferable examples of the sweetening agent include saccharin sodium, dipotassium glycyrrhizinate, aspartame, stevia and the like.

### (16) Dosage form

Examples of the dosage form of the pharmaceutical composition include oral agents such as tablet, capsule (including soft capsule and microcapsule), granule, powder, syrup, emulsion, suspension and the like; and parenteral agents such as injection (e.g., subcutaneous injection, intravenous injection, intramuscular injection, intraperitoneal injection etc.), external preparation (e.g., preparation for nasal administration, transdermal preparation, ointment etc.), suppository (e.g., rectal suppository, vaginal suppository etc.), pellet, drip infusion and sustained release preparation, and the like, all of which can be orally or parenterally administered safely.

The pharmaceutical composition can be produced according to a conventional method in the technical field of preparing medicine, such as the method described in Japan Pharmacopoeia and the like. A specific production method of a preparation is explained in detail in the following.

For example, oral agents are produced by adding, for example, excipient (e.g., lactose, sucrose, starch, D-mannitol etc.), disintegrant (e.g., carboxymethylcellulose calcium etc.), binder (e.g., pregelatinized starch, acacia, carboxymethylcellulose, hydroxypropylcellulose, polyvinylpyrrolidone etc.), lubricant (e.g., talc, magnesium stearate, polyethylene glycol 6000 etc.) and the like to the active ingredient, compression-molding the mixture, and where necessary, coating with a coating material for the purpose of masking taste, or affording enteric property or sustained release property by a method known *per se*.

Examples of the coating material include sugar coating material, water-soluble film coating material, enteric film coating material, sustained release film coating material and the like.

As the sugar coating material, sucrose is used, and one or more kinds selected from talc, precipitated calcium carbonate, gelatin, acacia, pullulan, carnauba wax and the like may be used in combination.

Examples of the water-soluble film coating material include cellulose polymers such as hydroxypropylcellulose, hydroxypropylmethylcellulose, hydroxyethylcellulose, methylhydroxyethylcellulose and the like; synthetic polymers such as polyvinylacetaldiethylaminoacetate, aminoalkylmethacrylate copolymer E [Eudragit E (trademark), Röhm Pharma], polyvinylpyrrolidone and the like; polysaccharides such as pullulan and the like; and the like.

Examples of the enteric film coating material include cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L [Eudragit L (trademark), Röhm Pharma], methacrylic acid copolymer LD [Eudragit L-30D55 (trademark), Röhm Pharma], methacrylic acid copolymer S [Eudragit S (trademark), Röhm Pharma] and the like; naturally occurring substances such as shellac and the like; and the like.

Examples of the sustained release film coating material include cellulose polymers such as ethylcellulose and the like; acrylic acid polymers such as aminoalkylmethacrylate copolymer RS [Eudragit RS (trade name), Röhm Pharma], ethyl acrylate-methyl methacrylate copolymer suspension [Eudragit NE (trade name), Röhm Pharma] and the like; and the like.

The above-mentioned coating materials may be used in combination of two or more kinds thereof admixed at a suitable proportion. For coating, for example, shading agents such as titanium oxide, red ferric oxide and the like may be used.

Injections are produced by dissolving, suspending or emulsifying the active ingredient along with a dispersant (e.g., polysorbate 80, polyoxyethylene hydrogenated castor oil 60 etc.), polyethylene glycol, carboxymethylcellulose, sodium alginate and etc.), a antiseptic (e.g., methylparaben, propylparaben, benzyl alcohol, chlorobutanol, phenol etc.), an isotonicity agent (e.g., sodium chloride, glycerin, D-mannitol, D-sorbitol, glucose etc.) and the like in an aqueous solvent (e.g., distilled water, physiological saline, Ringer's solution etc.) or an oily solvent (e.g., plant oils such as olive oil, sesame oil, cotton seed oil, corn oil etc., propylene glycol etc.) and the like. Where desired, additives such as dissolution aid (e.g., sodium salicylate, sodium acetate etc.), stabilizer (e.g., human serum albumin etc.), soothing agent (e.g., benzyl alcohol etc.) and the like may be used.

### (17) Agent

The compound of the present invention can be used as an agent for improving insulin resistance, an agent for enhancing insulin sensitivity, an agent for regulating a retinoid-related receptor function, a ligand for a peroxisome proliferator-activated receptor, a retinoid X receptor ligand and the like. As used herein, by the function regulating agent is meant both an agonist and an antagonist. The function regulating agent may be a partial agonist or a partial antagonist, preferably an agonist.

The compound of the present invention has a hypoglycemic action, a hypolipidemic action, a hypoinsulinemic action, an insulin resistance improving action, an insulin sensitivity enhancing action, and a retinoid-related receptor function regulating action. By the "retinoid-related receptor" here is meant a DNA-binding transcription factor included in the nuclear receptors, and whose ligand is a signal molecule such as oil-soluble vitamins and the like. It may be any of a monomer receptor, a homodimer receptor and a heterodimer receptor.

Here, examples of the monomer receptor include retinoid O receptor (hereinafter also abbreviated as ROR) α (GenBank Accession No. L14611), RORβ (GenBank Accession No. L14160), RORγ (GenBank Accession No. U16997); Rev-erb α (GenBank Accession No. M24898), Rev-erb β (GenBank Accession No. L31785); ERRα (GenBank Accession No. X51416), ERRβ (GenBank Accession No. X51417); Ftz-FIα (GenBank Accession No. S65876), Ftz-FIβ (GenBank Accession No. M81385); TIx (GenBank Accession No. S77482); GCNF (GenBank Accession No. U14666); and the like.

Examples of the homodimer receptor include homodimers formed from retinoid X receptor (hereinafter, also abbreviated as RXR) α (GenBank Accession No. X52773), RXRβ (GenBank Accession No. M84820), RXRγ (GenBank Accession No. U38480); COUPα (GenBank Accession No. X12795), COUPβ (GenBank Accession No. M64497), COUPγ (GenBank Accession No. X12794); TR2α (GenBank Accession No. M29960), TR2β (GenBank Accession No. L27586); or HNF4α (GenBank Accession No. X76930), HNF4γ (GenBank Accession No. Z49826), and the like.

Examples of the heterodimer receptor include heterodimers which are formed from the above-mentioned retinoid X receptor (RXRα, RXRβ or RXRγ) and one receptor selected from retinoid A receptor (hereinafter, also abbreviated as RAR) α (GenBank Accession No. X06614), RARβ (GenBank Accession No. Y00291), RARγ (GenBank Accession No. M24857); thyroid hormone receptor (hereinafter, also abbreviated as TR) α (GenBank Accession No. M24748), TRβ (GenBank Accession No. M26747); vitamin D receptor (VDR) (GenBank Accession No. J03258): peroxisome proliferator-activated receptor (hereinafter, also abbreviated as PPAR) α (GenBank Accession No. L02932), PPARβ (PPAR δ) (GenBank Accession No. U10375), PPAR γ (GenBank Accession No. L40904); LXRα (GenBank Accession No. U22662), LXRβ (GenBank Accession No. U14534); FXR (GenBank Accession. No. U18374); MB67 (GenBank Accession No. L29263); ONR (GenBank Accession No. X75163); and NURα (GenBank Accession No. L13740), NURβ (GenBank Accession No. X75918) and NURγ (GenBank Accession No. U12767).

The compound of the present invention has an excellent ligand activity particularly to retinoid X receptors (RXRα, RXRβ, RXRγ) and to peroxisome proliferator-activated receptors (PPARα, PPARβ (PPARδ), PPARγ), from among the above-mentioned retinoid-related receptors.

Further, the compound of the present invention has an excellent ligand activity to peroxisome proliferator-activated receptors in heterodimer receptors formed from a retinoid X receptor and a peroxisome proliferator-activated receptor, and preferably in heterodimer receptors formed from RXRα and PPARγ.

Accordingly, the retinoid-related receptor ligand of the present invention can be used suitably as a ligand for peroxisome proliferator-activated receptors or a ligand for retinoid X receptors.

### (18) Target disease

The compound of the present invention and the pharmaceutical composition of the present invention can be used as an agent for the prophylaxis or treatment of diabetes (e.g., type I diabetes, type II diabetes, gestational diabetes and the like); an agent for the prophylaxis or treatment of hyperlipidemia (e.g., hypertriglyceridemia, hypercholesterolemia, hypo-high-density-lipoproteinemia, postprandial hyperlipidemia and the like); an agent for improving insulin resistance; an agent for enhancing insulin sensitivity; an agent for the prophylaxis or treatment of impaired glucose tolerance (IGT); and an agent for preventing progress from impaired glucose tolerance to diabetes.

For diagnostic criteria of diabetes, Japan Diabetes Society reported new diagnostic criteria in 1999.

According to this report, diabetes is a condition showing any of a fasting blood glucose level (glucose concentration in venous plasma) of not less than 126 mg/dl, a 75 g oral glucose tolerance test (75 g OGTT) 2 h level (glucose concentration in venous plasma) of not less than 200 mg/dl, a non-fasting blood glucose level (glucose concentration in venous plasma) of not less than 200 mg/dl. A condition not falling under the above-mentioned diabetes and not being "a condition showing a fasting blood glucose level (glucose concentration in venous plasma) of less than 110 mg/dl or a 75 g oral glucose tolerance test (75 g OGTT) 2 h level (glucose concentration in venous plasma) of less than 140 mg/dl" (normal type) is called a "borderline type".

In addition, ADA (American Diabetes Association) reported new diagnostic criteria of diabetes in 1997 and WHO in 1998.

According to these reports, moreover, diabetes is a condition showing a fasting blood glucose level (glucose concentration in venous plasma) of not less than 126 mg/dl and a 75 g oral glucose tolerance test 2 h level (glucose concentration in venous plasma) of not less than 200 mg/dl.

According to the above-mentioned reports, impaired glucose tolerance is a condition showing a fasting blood glucose level (glucose concentration in venous plasma) of less than 126 mg/dl and a 75 g oral glucose tolerance test 2 h level (glucose concentration in venous plasma) of not less than 140 mg/dl and less than 200 mg/dl. According to the report of ADA, a condition showing a fasting blood glucose level (glucose concentration in venous plasma) of not less than 110 mg/dl and less than 126 mg/dl is called IFG (Impaired Fasting Glucose). According to the report of WHO, among the IFG (Impaired Fasting Glucose), a condition showing a 75g oral glucose tolerance test 2 h level (glucose concentration in venous plasma) of less than 140 mg/dl is called IFG (Impaired Fasting Glycemia).

The compound of the present invention and the pharmaceutical composition of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention and the pharmaceutical composition of the present invention can prevent progress of borderline type, impaired glucose tolerance, IFG (Impaired Fasting Glucose) or IFG (Impaired Fasting Glycemia) into diabetes.

The compound of the present invention and the pharmaceutical composition of the present invention can be also used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infections, inferior limb infection and the like), diabetic gangrene, xerostomia, hypacusis, cerebrovascular disorder, peripheral blood circulation disorder and the like], obesity, osteoporosis, cachexia (e.g., cancerous cachexia, tuberculous cachexia, diabetic cachexia, hemopathic cachexia, endocrinopathic cachexia, infectious cachexia, cachexia due to acquired immunodeficiency syndrome), fatty liver, hypertension, polycystic ovary syndrome, renal diseases (e.g., diabetic nephropathy, glomerular nephritis, glomerulosclerosis, nephrotic syndrome, hypertensive nephrosclerosis, terminal renal disease and the like), muscular dystrophy, myocardial infarction, angina pectoris, cerebrovascular disorders (e.g., cerebral infarction, cerebral apoplexy), insulin resistance syndrome, syndrome X, hyperinsulinemia, hyperinsulinemia-induced sensory disorder, tumors (e.g., leukemia, breast cancer, prostate cancer, skin cancer and the like), irritable bowel syndrome, acute or chronic diarrhea, visceral obesity syndrome and the like.

Also, the compound of the present invention and the pharmaceutical composition of the present invention can be used for ameliorating the condition of bellyache, nausea, vomiting, dysphoria in epigastrium and the like, each of which is accompanied by peptic ulcer, acute or chronic gastritis, biliary dyskinesia, cholecystitis and the like.

Further, the compound of the present invention and the pharmaceutical composition of the present invention can control (enhance or inhibit) appetite, and therefore, can be used as an agent for treating, for example, leanness and cibophobia (the weight increase in administration subjects suffering from leanness or cibophobia) or an agent for treating obesity.

Of the compounds of the present invention, compound (I) can be used as an agent for the prophylaxis or treatment of inflammatory diseases (e.g., rheumatoid arthritis, spondylitis deformans, osteoarthritis, lumbago, gout, postoperative or traumatic inflammation, remission of swelling, neuralgia, laryngopharyngitis, cystitis, hepatitis (including non-alcoholic steatohepatitis), pneumonia, pancreatitis, inflammatory bowel disease, ulcerative colitis and the like), and the like. The compound (I) possesses a total cholesterol lowering action and enhances a plasma anti-arteriosclerosis index [(HDL cholesterol/total cholesterol)×100], and therefore, can be used as an agent for the prophylaxis or treatment of arteriosclerosis (e.g., atherosclerosis and the like), and the like.

The compound of the present invention and the pharmaceutical composition of the present invention can be also used as an agent for the prophylaxis or treatment of TNF-α mediated inflammatory diseases. Here, the TNF-α mediated inflammatory diseases mean those that occur due to the presence of TNF-α and can be treated by way of a TNF-α inhibitory action. Examples of such inflammatory diseases include diabetic complications (e.g., retinopathy, nephropathy, neuropathy, macroangiopathy and the like), rheumatoid arthritis, spondylitis deformans, osteoarthritis, lumbago, gout, postoperative or traumatic inflammation, remission of swelling, neuralgia, pharyngolaryngitis, cystitis, hepatitis, pneumonia, gastric mucosal injury (including aspirin-induced gastric mucosal injury), and the like.

The compound of the present invention and the pharmaceutical composition of the present invention have an apoptosis inhibitory action, and can be used as an agent for the prophylaxis and treatment of diseases mediated by promotion of apoptosis. Here, examples of the diseases mediated by promotion of apoptosis include viral diseases (e.g., AIDS, fulminant hepatitis and the like), neurodegenerative diseases (e.g., Alzheimer's disease, Parkinson's disease, amyotropic lateral sclerosis, retinitis pigmentosa, cerebellar degeneration and the like), myelodysplasia (e.g., aplastic anemia and the like), ischemic diseases (e.g., myocardial infarction, cerebral apoplexy and the like), hepatic diseases (e.g., alcoholic hepatitis, hepatitis B, hepatitis C and the like), joint-diseases (e.g., osteoarthritis and the like), atherosclerosis, and the like.

The compound of the present invention and the pharmaceutical composition of the present invention can be also used for decreasing visceral fat, suppressing visceral fat accumulation, improving glycometabolism, improving lipid metabolism, improving insulin resistance, suppressing production of oxidized LDL, improving lipoprotein metabolism, improving coronary artery metabolism, prophylaxis and treatment of cardiovascular complications, prophylaxis and treatment of heart failure complications, lowering blood remnant, prophylaxis and treatment of anovulation, prophylaxis and treatment of hypertrichosis, prophylaxis and treatment of hyperandrogenemia, and the like.

The compound of the present invention and the pharmaceutical composition of the present invention can be also used for secondary prophylaxis and suppression of progression of the above-mentioned various diseases (e.g., cardiovascular event such as myocardial infarction and the like).

The compound of the present invention and the pharmaceutical composition of the present invention can be also used in combination with midazolam, ketokonazole and the like.

The dose of the compound of the present invention and the pharmaceutical composition of the present invention can be appropriately determined depending on the administration subject, administration route, target disease, condition and the like. When, for example, the administration subject is adult human with diabetes, the compound of the present invention, which is an active ingredient, is orally administered generally in a single dose of about 0.01-100 mg/kg body weight, preferably 0.05-10 mg/kg body weight, more preferably 0.1-2 mg/kg body weight, which amount is desirably given once to three times a day.

### (19) Combined use of medicaments

The compound of the present invention can be used in combination with therapeutic agents such as a therapeutic agent of diabetes, a therapeutic agent of diabetic complications, an antihyperlipidemia agent, an antihypertensive agent, an antiobestic agent, a diuretic, a chemotherapeutic agent, an immunotherapeutic agent, a therapeutic agent of osteoporosis, an antidementia agent, an agent for improving erectile dysfunction, a therapeutic agent of incontinentia and/or pollakiuria and the like (hereinafter to be referred to as a combination drug). In this case, the timing of administration of the compound of the present invention and a combination drug is not limited. These may be simultaneously administered to an administration object or administered in a staggered manner. The dose of the combination drug can be determined as appropriate based on the dose clinically employed. The proportion of the compound of the present invention and combination drug can be appropriately determined depending on the administration subject, administration route, target disease, condition, combination and the like. When, for example, the administration subject is human, a combination drug is preferably used in an amount of 0.01-100 parts by weight per 1 part by weight of the compound of the present invention.

Examples of the therapeutic agent of diabetes include insulin preparations (e.g., animal insulin preparations extracted from pancreas of cattle, swine; human insulin preparations synthesized by genetic engineering techniques using *Escherichia coli* or yeast, and the like; insulin sensitizers (e.g., pioglitazone hydrochloride, troglitazone, rosiglitazone, GI-262570, JTT-501, MCC-555, YM-440, KRP-297, CS-011, FK-614 and the like), α-glucosidase inhibitors (e.g., voglibose, acarbose, miglitol, emiglitate and the like), biguanide agents (e.g., phenformin, metformin, buformin and the like), insulin secretagogues [e.g., sulfonylurea agents (e.g., tolbutamide, glibenclamide, gliclazide, chlorpropamide, tolazamide, acetohexamide, glyclopyramide, glimepiride, glipizide, glybuzole and the like), repaglinide, senaglinide, nateglinide, mitiglinide or calcium salt hydrate thereof, GLP-1 and the like], amyrin agonists (e.g., pramlintide and the like), phosphotyrosine phosphatase inhibitors (e.g., vanadic acid and the like), dipeptidylpeptidase IV inhibitors (e.g., NVP-DPP-278, PT-100, P32/98 and the like), β3 agonists (e.g., CL-316243, SR-58611-A, UL-TG-307, SB-226552, AJ-9677, BMS-196085, AZ40140 and the like), gluconeogenesis inhibitors (e.g., glycogen phosphorylase inhibitors, glucose-6-phosphatase inhibitors, glucagon antagonists and the like), SGLT (sodium-glucose cotransporter) inhibitors (e.g., T-1095 and the like) and the like.

Examples of the therapeutic agent of diabetic complications include aldose reductase inhibitors (e.g., Tolrestat, Epalrestat, Zenarestat, Zopolrestat, Minalrestat, Fidarestat, SNK-860, CT-112 and the like), neurotrophic factors (e.g., NGF, NT-3, BDNF and the like), PKC inhibitors (e.g., LY-333531 and the like), AGE inhibitors (e.g., ALT946, pimagedine, pyratoxanthine, N-phenacylthiazolium bromide (ALT766), EXO-226 and the like), active oxygen scavengers (e.g., thioctic acid and the like), cerebral vasodilators (e.g., tiapride, mexiletine and the like), and the like.

Examples of the antihyperlipidemia agent include statin compounds that are cholesterol synthesis inhibitors (e.g., cerivastatin, pravastatin, simvastatin, lovastatin, atorvastatin, fluvastatin, itavastatin and salts thereof (e.g., sodium salt) and the like), squalene synthase inhibitors or fibrate compounds having a hypotriglyceridemic action (e.g., bezafibrate, clofibrate, simfibrate, clinofibrate and the like), and the like.

Examples of the antihypertensive agent include angiotensin converting enzyme inhibitors (e.g., captopril, enalapril, delapril and the like), angiotensin II antagonists (e.g., candesartan cilexetil, losartan, eprosartan, valsartan, telmisartan, irbesartan, tasosartan and the like), calcium antagonists (e.g., manidipine, nifedipine, amlodipine, efonidipine, nicardipine and the like), Clonidine and the like.

Examples of the antiobestic agent include central antiobestic agents (e.g., Dexfenfluramine, fenfluramine, phentermine, Sibutramine, amfepramone, dexamphetamine, Mazindol, phenylpropanolamine, clobenzorex and the like), pancreatic lipase inhibitors (e.g., orlistat and the like), β3 agonists (e.g., CL-316243, SR-58611-A, UL-TG-307, SB-226552, AJ-9677, BMS-196085, AZ40140 and the like), peptide anorexiants (e.g., leptin, CNTF (Ciliary Neurotropic Factor) and the like), cholecystokinin agonists (e.g., lintitript, FPL-15849 and the like) and the like.

Examples of the diuretic include xanthine derivatives (e.g., sodium salicylate and theobromine, calcium salicylate and theobromine and the like), thiazide preparations (e.g., ethiazide, cyclopenthiazide, trichloromethyazide, hydrochlorothiazide, hydroflumethiazide, benzylhydrochlorothiazide, penflutizide, polythiazide, methyclothiazide and the like), antialdosterone preparations (e.g., spironolactone, triamterene and the like), carbonate dehydratase inhibitors (e.g., acetazolamide and the like), chlorobenzenesulfonamide preparations (e.g., chlortalidone, mefruside, indapamide and the like), azosemide, isosorbide, etacrynic acid, piretanide, bumetanide, furosemide and the like.

Examples of the chemotherapeutic agent include alkylation agents (e.g., cyclophosphamide, ifosfamide and the like), metabolic antagonists (e.g., methotrexate, 5-fluorouracil and the like), anti-cancer antibiotics (e.g., mitomycin, adriamycin and the like), plant-derived anti-cancer agents (e.g., vincristin, vindesine, taxol and the like), cisplatin, carboplatin, etopoxide and the like. Of these, furtulon and neofurtulon which are 5-fluorouracil derivatives and the like are preferable.

Examples of the immunotherapeutic agent include microorganism or bacterial components (e.g., muramyl dipeptide derivative, picibanil and the like), polysaccharides having immunity potentiating activity (e.g., lentinan, sizofiran, krestin and the like), cytokines obtained by genetic engineering techniques (e.g., interferon, interleukin (IL) and the like), colony stimulating factors (e.g., granulocyte colony stimulating factor, erythropoietin and the like) and the like, with preference given to IL-1, IL-2, IL-12 and the like.

Examples of the therapeutic agent of osteoporosis include alfacalcidol, calcitriol, elcaltonin, calcitonin salmon, estriol, ipriflavone, pamidronate disodium, alendronate sodium hydrate, incadronate disodium and the like.

Examples of the antidementia agent include tacrine, donepezil, rivastigmine, galantamine and the like.

Examples of the agent for improving erectile dysfunction include apomorphine, sildenafil citrate and the like.

Examples of the therapeutic agent of incontinentia and/or pollakiuria include flavoxate hydrochloride, oxybutynin hydrochloride, propiverine hydrochloride and the like.

Furthermore, drugs having a cachexia-improving action established in animal models and clinical situations, such as cyclooxygenase inhibitors (e.g., Indometacin and the like) [Cancer Research, vol. 49, 5935-5939, 1989], Progesterone derivatives (e.g., Megesterol acetate) [Journal of Clinical Oncology, vol. 12, 213-225, 1994], glucosteroid (e.g., dexamethasone and the like), metoclopramide agents, tetrahydrocannabinol agents (*ibid*.), fat metabolism improving agents (e.g., eicosapentaenoic acid and the like) [British Journal of Cancer, vol. 68, 314-318, 1993], growth hormones, IGF-1, or antibodies to a cachexia-inducing factor such as TNF-α, LIF, IL-6, Oncostatin M, and the like can be used in combination with the compound of the present invention.

The combination drug is preferably an insulin preparation, an insulin sensitizer, an α-glucosidase inhibitor, a biguanide agent, an insulin secretagogue (preferably a sulfonylurea agent) or the like.

Two or more of the above-mentioned combination drugs can be used in combination in an appropriate ratio. Preferable combinations in the case of using two or more combination drugs are, for example, as shown in the following.
1) Insulin sensitizer and insulin preparation;
2) Insulin sensitizer and insulin secretagogue (preferably sulfonylurea agent);
3) Insulin sensitizer and α-glucosidase inhibitor;
4) Insulin sensitizer and biguanide agent;
5) Insulin sensitizer, insulin preparation and biguanide agent;
6) Insulin sensitizer, insulin preparation and insulin secretagogue (preferably sulfonylurea agent);
7) Insulin sensitizer, insulin preparation and α-glucosidase inhibitor;
8) Insulin sensitizer, insulin secretagogue (preferably sulfonylurea agent) and biguanide agent;
9) Insulin sensitizer, insulin secretagogue (preferably sulfonylurea agent) and α-glucosidase inhibitor; and
10) Insulin sensitizer, biguanide agent and α-glucosidase inhibitor.

When the compound of the present invention or the pharmaceutical composition of the present invention is used in combination with a combination drug, the amount thereof can be reduced within a safe range in consideration of adverse effects of these agents. Particularly, the dose of an insulin sensitizer , an insulin secretagogue (preferably a sulfonylurea agent) and a biguanide agent can be reduced as compared with the normal dose. Therefore, an adverse effect which may be caused by these agents can be prevented safely. In addition, the dose of the agent for diabetic complications, antihyperlipidemia agent and antihypertensive agent can be reduced whereby an adverse effect which may be caused by these agents can be prevented effectively.

### (20) Production method

The production methods of the compound of the present invention are explained in the following. Since compound (I) is included in compound (II), the production method of compound (II) is explained.

The compound (II) of the present invention can be produced according to a method known *per se*, such as Method A to Method F described in the following, or an analogous method thereto.

### [Method A]

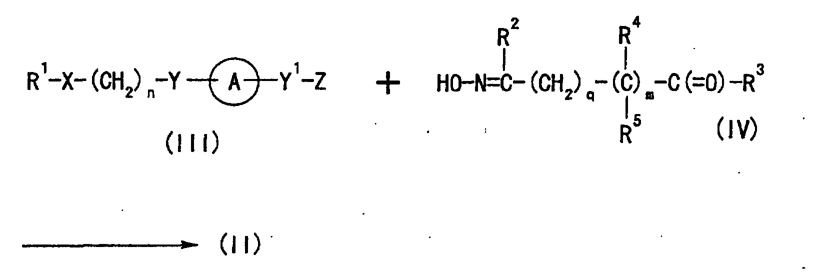

wherein Z is a hydroxy group, a halogen atom or a group represented by OSO₂R¹⁶ (R¹⁶ is alkyl group having 1 to 4 carbon atoms, aryl group having 6 to 10 carbon atoms optionally substituted by alkyl group having 1 to 4 carbon atoms, and other symbols are as defined above.

The alkyl group having 1 to 4 carbon atoms of the "alkyl group having 1 to 4 carbon atoms" and "aryl group having 6 to 10 carbon atoms optionally substituted by alkyl group having 1 to 4 carbon atoms" represented by R¹⁶ is exemplified by methyl, ethyl, propyl, isopropyl, butyl, isobutyl, sec.-butyl and t.-butyl, preferably methyl.

The aryl group having 6 to 10 carbon atoms of the "aryl group having 6 to 10 carbon atoms optionally substituted by alkyl group having 1 to 4 carbon atoms" represented by R¹⁶ is exemplified by phenyl and naphthyl, preferably phenyl.

According to this method, compound (III) is reacted with compound (IV) to produce compound (II).

When Z is a hydroxy group, this reaction is carried out by a method known *per se*, such as a method described in Synthesis, page 1 (1981), or an analogous method thereto. That is, this reaction is generally carried out in the presence of an organic phosphorus compound and electrophile in a solvent that does not adversely influence the reaction.

Examples of the organic phosphorus compound include triphenylphosphine, tributylphosphine and the like.

Examples of the electrophile include diethyl azodicarboxylate, diisopropyl azodicarboxylate, azodicarbonyldipiperazine and the like.

The amount of the organic phosphorus compound and electrophile to be used is preferably 1 to 5 molar equivalents per compound (IV).

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; and the like. These solvents may be used upon mixing at a suitable ratio.

The reaction temperature is generally -50°C to 150°C, preferably -10°C to 100°C.

The reaction time is generally from 0.5 to 20 hours.

When Z is a halogen atom or a group represented by OSO₂R¹⁶, this reaction is carried out according to a conventional method in the presence of a base in a solvent that does not adversely influence the reaction.

Examples of the base include alkali metal salts such as potassium hydroxide, sodium hydroxide, sodium hydrogencarbonate, potassium carbonate and the like; amines such as pyridine, triethylamine, N,N-dimethylaniline, 1,8-diazabicyclo[5.4.0]undeca-7-ene and the like; metal hydrides such as potassium hydride, sodium hydride and the like; alkali metal alkoxides such as sodium methoxide, sodium ethoxide, potassium t.-butoxide and the like; and the like.

The amount of the base to be used is preferably 1 to 5 molar equivalents per compound (IV).

Examples of the solvent that does not adversely influence the reaction include aromatic hydrocarbons such as benzene, toluene, xylene and the like; ethers such as tetrahydrofuran, dioxane and the like; ketones such as acetone, 2-butanone and the like; halogenated hydrocarbons such as chloroform, dichloromethane and the like; amides such as N,N-dimethylformamide and the like; sulfoxides such as dimethyl sulfoxide and the like; and the like. These solvents may be used upon mixing at a suitable ratio.

The reaction temperature is generally -50°C to 150°C, preferably -10°C to 100°C.

The reaction time is generally from 0.5 to 20 hours.

The thus-obtained compound (II) can be isolated and purified by a known separation and purification means, such as concentration, concentration under reduced pressure, solvent extraction, crystallization, recrystallization, phase transfer, chromatography and the like.

The compound (III) and compound (IV) used as a starting compound in the above-mentioned Method A are known compounds and, for example, compound (III) wherein Z is a hydroxy group is described in EP-A 710659. Alternatively, the compound (III) is described in EP-A 629624 (JP-A-7-53555), WO 98/03505 and the like. The compound (III) can be also produced according to methods analogous to the methods described in these publications.

The compound (IV) is described in, for example, Journal fur Praktische Chemie, vol. 311, p. 370 (1969); Canadian Journal of Chemistry, vol. 48, p. 1948 (1970); Journal of Heterocyclic Chemistry, vol. 25, p. 1283 (1988) and the like. The compound (IV) can be also produced according to the methods analogous to the methods described in these documents.

Compound (II-1) having a phenyl substituted by an optionally substituted aliphatic hydrocarbon group and the like for R² in the formula (II), is also produced according to the following Method B.

### [Method B]

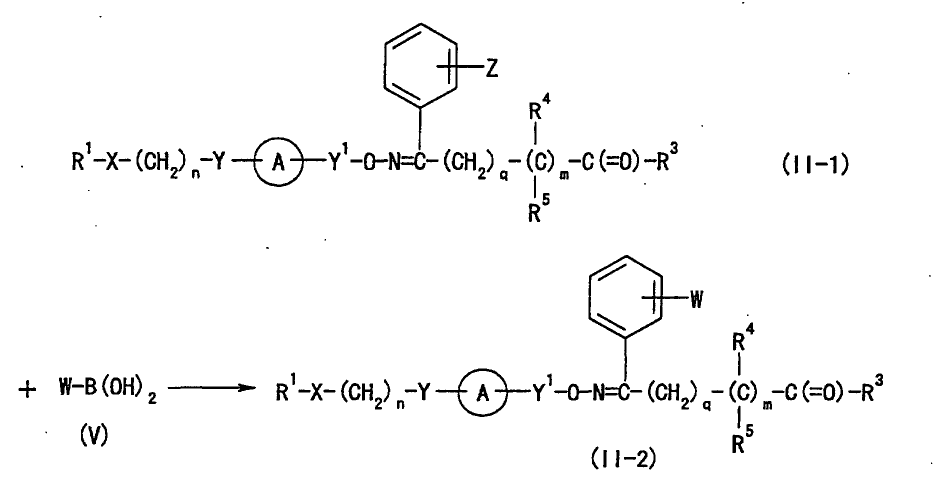

wherein W is an optionally substituted aliphatic hydrocarbon group, optionally substituted aromatic hydrocarbon group or optionally substituted aromatic heterocyclic group, and other symbols are as defined above.

The "optionally substituted aliphatic hydrocarbon group, optionally substituted aromatic hydrocarbon group or optionally substituted aromatic heterocyclic group" represented by W includes, for example, the "optionally substituted aliphatic hydrocarbon group", "optionally substituted aromatic hydrocarbon group" and "optionally substituted aromatic heterocyclic group" exemplified for the substituent in R¹.

According to this method, compound (II-1) is reacted with a boronic acid compound (V) to produce compound (II-2).

This reaction is carried out according to a method known *per se*, such as a method described in Journal of Organic Chemistry, vol. 58, p. 2201 (1993) or Journal of Organic Chemistry, vol. 60, p. 1060 (1995), in the presence of a metal catalyst and a base and in a solvent that does not adversely influence the reaction.

As the metal catalyst, for example, palladium(0), nickel(0) and the like are mentioned. Here, the palladium(0) catalyst is, for example, tris(dibenzylideneacetone)dipalladium, tetrakis(triphenylphosphine)palladium and the like, and the nickel (0) catalyst is, for example, 1,1'-bis(diphenylphosphino)ferrocene nickel and the like.

Examples of the base include alkali metal hydrogen carbonates such as sodium hydrogen carbonate and the like; alkaline metal carbonate such as sodium carbonate, potassium carbonate and the like; alkaline metal phosphate such as tripotassium phosphate and the like; and the like.

The amount of the metal catalyst to be used is, for example, 0.01-1 molar equivalent, preferably 0.05-0.5 molar equivalent, per compound (II-1).

The amount of the base to be used is, for example, 1-20 molar equivalents, preferably 1-10 molar equivalents, per compound (II-1).

Examples of the solvent that does not adversely influence the reaction include aromatic hydrocarbons such as benzene, toluene, and the like; alcohols such as methanol, ethanol and the like; ethers such as tetrahydrofuran, dioxane, and the like; water; and the like. These solvents may be used upon mixing at a suitable ratio. The kind of the solvent is determined as appropriate depending on the kind of metal catalyst.

The amount of the boronic acid compound (V) to be used is, for example, 1-7 molar equivalents, preferably 1-5 molar equivalents, per compound (II-1).

The reaction temperature is generally -20°C to 150°C, preferably 0°C to 100°C.

The reaction time is generally from 0.1 to 24 hours.

The compound (II-2) thus obtained can be isolated and purified by a known separation and purification means, such as concentration, concentration under reduced pressure, solvent extraction, crystallization, recrystallization, phase transfer, chromatography and the like.

The compound (II-1) used as a starting compound in the above-mentioned Method B is produced according to, for example, the above-mentioned Method A. The compound (V) is a known compound described in, for example, Organic Synthesis, vol. 39, p. 3 (1959); Journal of American Chemical Society, vol. 94, p. 4370 (1972) and the like. The compound (V) can be also produced according to a method analogous to the method described in these documents.

The compound (II) can be also produced according to the following Method C or Method D.

### [Method C]

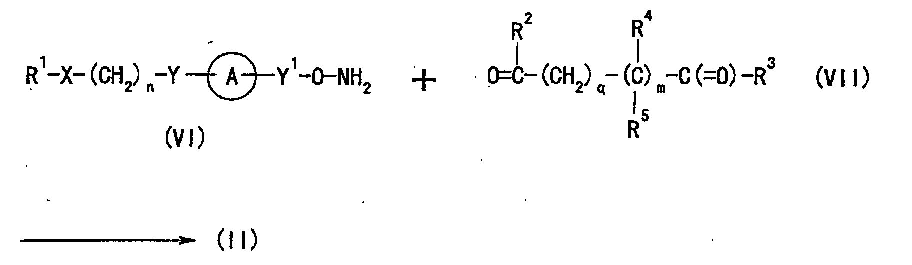

wherein the symbols in the formula are as defined above.

According to this method, compound (VI) is reacted with compound (VII) to produce compound (II). This reaction is carried out according to a method known *per se*.

This reaction is carried out, for example, in the presence of an acid or a base and in a solvent that does not influence the reaction.

As the acid, for example, hydrochloric acid, sulfuric acid, p-toluenesulfonic acid and the like can be mentioned. As the base, for example, sodium carbonate, potassium carbonate, sodium acetate, aq. ammonia and the like can be mentioned. The amount of the acid or base to be used is generally 1-10 molar equivalents per compound (VI).

Examples of the solvent that does not influence the reaction include ethers such as tetrahydrofuran, dioxane and the like; alcohols such as methanol, ethanol and the like; dimethyl sulfoxide; acetic acid; water; and the like. These solvents may be used upon mixing at a suitable ratio.

The reaction temperature is generally -50°C to 150°C, preferably -10°C to 120°C.

The reaction time is generally from 0.1 to 24 hours.

The thus-obtained compound (II) can be isolated and purified by a known separation and purification means, such as concentration, concentration under reduced pressure, solvent extraction, crystallization, recrystallization, phase transfer, chromatography and the like.

The compound (VI) used as a starting compound in the above-mentioned Method C can be produced according to a method known *per se,* such as a method described in, for example, Journal of Organic Chemistry, vol. 36, p. 3836 (1971) and the like, or a method analogous thereto.

### [Method D]

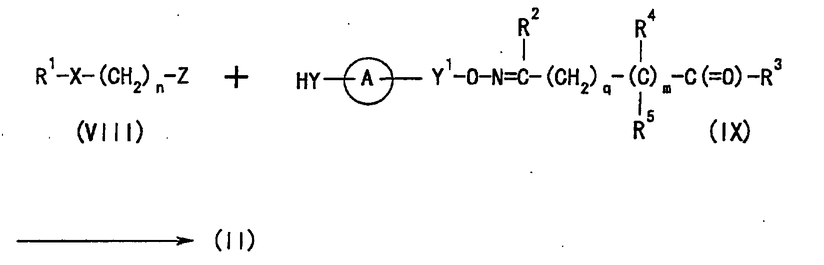

wherein the symbols in the formula are as defined above.

According to this method, compound (VIII) is reacted with compound (IX) to produce compound (II). This reaction is carried out in the same manner as in the reaction between compound (III) and compound (IV) in Method A.

The thus-obtained compound (II) can be isolated and purified by a known separation and purification means, such as concentration, concentration under reduced pressure, solvent extraction, crystallization, recrystallization, phase transfer, chromatography and the like.

The compound (IX) used as a starting compound in the above-mentioned Method D can be produced according to, for example, the following Method G.

### [Method G]

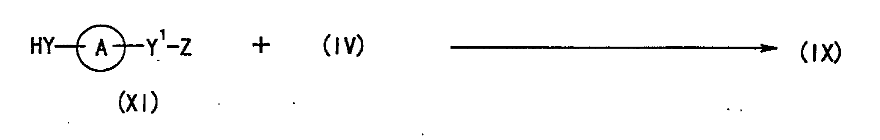

wherein the symbols in the formula are as defined above.

This method is carried out in the same manner as in the reaction between compound (III) and compound (IV) in Method A. The -YH moiety of compound (XI) may be protected before condensation reaction and deprotected after the reaction. Examples of the protecting group include benzyl group, methoxymethyl group, silyl group (e.g., trimethylsilyl group, t.-butyldimethylsilyl group and the like) and the like.

Compound (II-4) having OH for R³ in the formula (II) is produced according to, for example, the following Method E.

### [Method E]

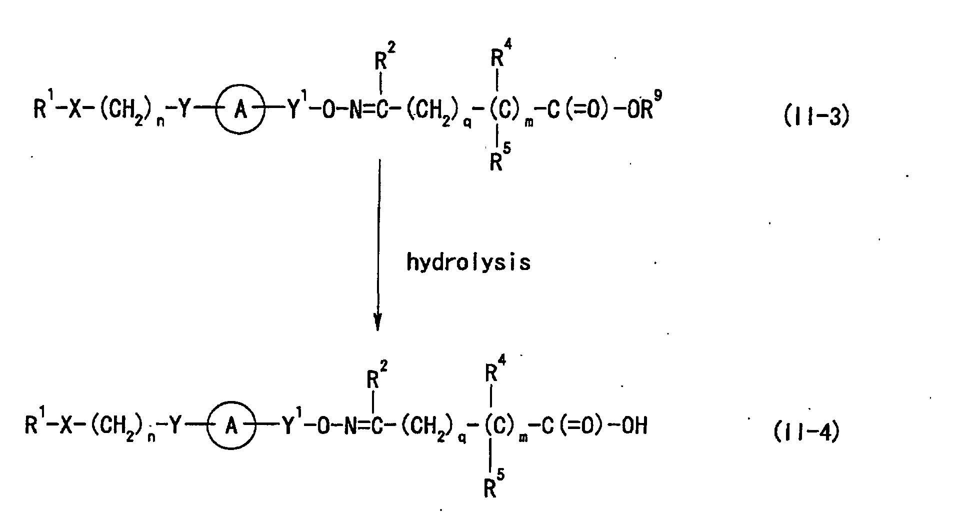

wherein the symbols in the formula are as defined above.

According to this method, compound (II-3) is hydrolyzed to produce compound (II-4).

This hydrolysis reaction is carried out in an aqueous solvent in the presence of an acid or a base according to a conventional method.

As the acid, for example, hydrochloric acid, sulfuric acid, acetic acid, hydrobromic acid and the like are mentioned.

Examples of the base include alkaline metal carbonates such as potassium carbonate, sodium carbonate and the like; alkaline metal alkoxides such as sodium methoxide and the like; alkaline metal hydroxides such as potassium hydroxide, sodium hydroxide, lithium hydroxide and the like; and the like.

The amounts of the acid and base to be used are generally excess amounts relative to compound (II-3). Preferably, the amount of the acid to be used is 2 to 50 equivalents and the amount of the base to be used is 1.2 to 5 equivalents, both per compound (II-3).

As the aqueous solvent, for example, a mixed solvent of water with one or more solvents selected from alcohols such as methanol, ethanol and the like; ethers such as tetrahydrofuran, dioxane and the like; dimethyl sulfoxide, acetone and the like, and the like can be mentioned.

The reaction temperature is generally -20°C to 150°C, preferably -10°C to 100°C.

The reaction time is generally from 0.1 to 20 hours.

The thus-obtained compound (II-4) can be isolated and purified by a known separation and purification means, such as concentration, concentration under reduced pressure, solvent extraction, crystallization, recrystallization, phase transfer, chromatography and the like.

The compound (II-3) used as a starting compound in the above-mentioned Method E is produced according to, for example, the above-mentioned Method A - Method D.

Compound (II-5) having NR⁹R¹⁰ for R³ in the formula (II) is produced according to, for example, the following Method F.

### [Method F]

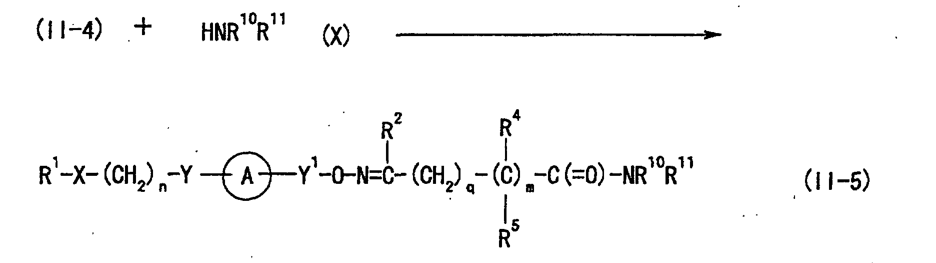

wherein the symbols in the formula are as defined above.

According to this method, compound (II-4) is amidated to produce compound (II-5). This reaction is carried out according to a method known per se, such as a method comprising directly condensing compound (II-4) with compound (X) using a condensing agent (e.g., dicyclohexylcarbodiimide and the like), a method comprising appropriately reacting a reactive derivative of compound (II-4) with compound (X) or other method. As used herein, the reactive derivative of compound (II-4) includes, for example, acid anhydride, acid halide (acid chloride, acid bromide), imidazolide, or mixed acid anhydride (e.g., anhydride of methyl carbonate, ethyl carbonate and/or isobutyl carbonate and the like) and the like.

When, for example, acid halide is used, the reaction is carried out in the presence of a base and in a solvent that does not influence the reaction.

Examples of the base include triethylamine, N-methylmorpholine, N,N-dimethylaniline, sodium hydrogencarbonate, sodium carbonate, potassium carbonate and the like.

Examples of the solvent that does not influence the reaction include halogenated hydrocarbons such as chloroform, dichloromethane and the like; aromatic hydrocarbons such as benzene, toluene and the like; ethers such as tetrahydrofuran, dioxane and the like; ethyl acetate, water and the like. These solvents may be used upon mixing at a suitable ratio.

The amount of compound (X) to be used is generally 1-10 molar equivalents, preferably 1-3 molar equivalents, per compound (II-4).

The reaction temperature is generally -30°C to 100°C.

The reaction time is from 0.5 to 20 hours.

When a mixed acid anhydride is used, compound (II-4) is reacted with chlorocarbonate (e.g., methyl chlorocarbonate, ethyl chlorocarbonate, isobutyl chlorocarbonate and the like) in the presence of a base (e.g., triethylamine, N-methylmorpholine, N,N-dimethylaniline, sodium hydrogencarbonate, sodium carbonate, potassium carbonate and the like) and then reacted with compound (X).

The amount of compound (X) to be used is generally 1-10 molar equivalents, preferably 1-3 molar equivalents, per compound (II-4).

The reaction temperature is generally -30°C to 100°C.

The reaction time is generally from 0.5 to 20 hours.

The thus-obtained compound (II-5) can be isolated and purified by a known separation and purification means, such as concentration, concentration under reduced pressure, solvent extraction, crystallization, recrystallization, phase transfer, chromatography and the like.

In each of the aforementioned reactions, when the starting compound has amino, carboxy, hydroxy or carbonyl as a substituent, such group may be protected by a protecting group generally used in the peptide chemistry and the like. The protecting group can be removed after reaction as necessary to give the objective compound.

As the amino-protecting group, for example, formyl, C₁₋₆ alkyl-carbonyl (e.g., acetyl, propionyl and the like), C₁₋₆ alkoxy-carbonyl (e.g., methoxycarbonyl, ethoxycarbonyl, tert-butoxycarbonyl and the like), benzoyl, C₇₋₁₀ aralkyl-carbonyl (e.g., benzylcarbonyl and the like), C₇₋₁₄ aralkyloxy-carbonyl (e.g., benzyloxycarbonyl, 9-fluorenylmethoxycarbonyl and the like), trityl, phthaloyl, N,N-dimethylaminomethylene, silyl (e.g., trimethylsilyl, triethylsilyl, dimethylphenylsilyl, tert-butyldimethylsilyl, tert-butyldiethylsilyl and the like), C₂₋₆ alkenyl (e.g., 1-allyl and the like) and the like are mentioned. These groups are optionally substituted by 1 to 3 halogen atoms (e.g., fluorine, chlorine, bromine, iodine and the like), C₁₋₆ alkoxy (e.g., methoxy, ethoxy, propoxy and the like), nitro and the like.

Examples of the carboxy-protecting group include C₁₋₆ alkyl (e.g., methyl, ethyl, propyl, isopropyl, butyl, tert-butyl and the like), C₇₋₁₁ aralkyl (e.g., benzyl and the like), phenyl, trityl, silyl (e.g., trimethylsilyl, triethylsilyl, dimethylphenylsilyl, tert-butyldimethylsilyl, tert-butyldiethylsilyl and the like), C₂₋₆ alkenyl (e.g., 1-allyl and the like) and the like. These groups are optionally substituted by 1 to 3 halogen atoms (e.g., fluorine, chlorine, bromine, iodine and the like), C₁₋₆ alkoxy (e.g., methoxy, ethoxy, propoxy and the like), nitro and the like.

Examples of the hydroxy-protecting group include C₁₋₆ alkyl (e.g., methyl, ethyl, propyl, isopropyl, butyl, tert-butyl and the like), phenyl, trityl, C₇₋₁₀ aralkyl (e.g., benzyl and the like), formyl, C₁₋₆ alkyl-carbonyl (e.g., acetyl, propionyl and the like), benzoyl, C₇₋₁₀ aralkyl-carbonyl (e.g., benzylcarbonyl and the like), 2-tetrahydropyranyl, 2-tetrahydrofuranyl, silyl (e.g., trimethylsilyl, triethylsilyl, dimethylphenylsilyl, tert-butyldimethylsilyl, tert-butyldiethylsilyl and the like), C₂₋₆ alkenyl (e.g., 1-allyl and the like) and the like. These groups are optionally substituted by 1 to 3 halogen atoms (e.g., fluorine, chlorine, bromine, iodine and the like), C₁₋₆ alkyl (e.g., methyl, ethyl, propyl and the like), C₁₋₆ alkoxy (e.g., methoxy, ethoxy, propoxy and the like), nitro and the like.

Examples of the carbonyl-protecting group include cyclic acetal (e.g., 1,3-dioxane and the like), acyclic acetal (e.g., di-C₁₋₆ alkylacetal and the like) and the like.

These protecting groups can be removed according to a method known *per se*, for example, the method described in Protective Groups in Organic Synthesis, John Wiley and Sons (1980) and the like. For example, a method using acid, base, ultraviolet radiation, hydrazine, phenylhydrazine, sodium N-methyldithiocarbamate, tetrabutylammoniumfluoride, palladium acetate, trialkylsilylhalide (e.g., trimethylsilyliodide, trimethylsilylbromide and the like) and the. like, a reduction method and the like can be employed.

When compound (II) contains an optical isomer, a stereoisomer, a positional isomer or a rotational isomer, these are also encompassed in compound (II), and can be obtained as a single product according to a synthetic method and separation method known *per se.* For example, when compound (II) has an optical isomer, an optical isomer resolved from this compound is also encompassed in compound (II).

The optical isomer can be produced by a method known *per se*. To be specific, an optically active synthetic intermediate is used, or the final racemate product is subjected to optical resolution according to a conventional method to give an optical isomer.

As the method of optical resolution, a method known *per se*, such as a fractional recrystallization method, a chiral column method, a diastereomer method and the like can be used.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine and the like) is formed, which is separated by a fractional recrystallization method, and a free optical isomer is obtained by a neutralization step where desired.

### 2) Chiral column method

A racemate or a salt thereof is applied to a column for separation of an optical isomer (chiral column) to allow separation. In the case of a liquid chromatography, for example, a mixture of an optical isomer is applied to a chiral column such as ENANTIO-OVM (manufactured by Tosoh Corporation) or CHIRAL series (manufactured by Daicel Chemical Industries, Ltd.) and the like, and then developed with water, various buffers (e.g., phosphate buffer) and organic solvents (e.g., ethanol, methanol, isopropanol, acetonitrile, trifluoroacetic acid, diethylamine and the like) solely or in a mixed solution to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemate mixture is converted to a diastereomeric mixture by chemical reaction with an optically active reagent, which is separated into a single substance by a typical separation means (e.g., fractional recrystallization, chromatography method and the like) and the like, and then subjected to a chemical treatment such as hydrolysis reaction and the like to cleave an optically active reagent moiety, whereby an optical isomer is obtained. For example, when compound (I) contains hydroxy, or primary or secondary amino in the molecule, the compound and an optically active organic acid (e.g., MPTA [α-methoxy-α-(trifluoromethyl)phenylacetic acid], (-)-menthoxyacetic acid and the like) and the like are subjected to condensation reaction to give an ester form or amide form diastereomer. When compound (I) has a carboxy group, this compound and an optically active amine or an alcohol reagent are subjected to condensation to give an amide form or ester form diastereomer. The separated diastereomer is converted to an optical isomer of the original compound by acid hydrolysis or base hydrolysis reaction.

### Best Mode for Embodying the Invention

The present invention is explained in more detail by the following Test Examples, Reference Examples, Examples and Formulation Examples, which are not to be construed as limitative. In the following Reference Examples and Examples, "%" means weight percent unless specifically indicated. The room temperature means a temperature of from 1°C to 30°C.

When a base, an amino acid and the like are expressed using abbreviations in the present specification, they are based on the abbreviations of IUPAC-IUB Commission on Biochemical Nomenclature or conventional abbreviations used in the art, which are exemplified by the following. When an amino acid has an optical isomer, it refers to an L form, unless specifically indicated.

In the Sequence Listing in the present specification, SEQ ID Nos. show the following sequences.
[SEQ ID No: 1] depicts the base sequence of primer PAG-U used in Reference Example 1.
[SEQ ID No: 2] depicts the base sequence of primer PAG-L used in Reference Example 1.
[SEQ ID No: 3] depicts the base sequence of primer XRA-U used in Reference Example 2.
[SEQ ID No: 4] depicts the base sequence of primer XRA-L used in Reference Example 2.
[SEQ ID No: 5] depicts the base sequence of PPRE-U used in Reference Example 4.
[SEQ ID Number: 6] depicts the base sequence of PPRE-L used in Reference Example 4.
[SEQ ID Number: 7] depicts the base sequence of primer TK-U used in Reference Example 4.
[SEQ ID Number: 8] depicts the base sequence of primer TK-L used in Reference Example 4.

### Examples

### Test Example 1

### Hypoglycemic and hypolipidemic (hypotriglyceridemic) actions in mice

Test compounds were mixed in a powdery diet (CE-2, Japan Clea) at the concentration of 0.01% (compound of Example 2) or 0.005% (compound of Example 4), and freely given to KKA^{y} mice (11 to 12 weeks old, 5 mice per group), a model of obesity and type II diabetes, for four days. During this period, water was given freely. Blood was sampled from orbital venous plexus, and glucose and triglyceride levels in plasma separated from blood were determined enzymatically using L type Wako Glu2 (Wako Pure Chemical Industries, Ltd.) or L type Wako TG•H (Wako Pure Chemical Industries, Ltd.), respectively. The results are given in Table 1.

In the Table, the value shows percent decrease (%) in the test compound administration group based on the value of the test compound non-administration group as 100%.

**Table 1**

| Test compound (Example No.) | Hypoglycemic action (%) | Hypolipidemic action (%) |
|---|---|---|
| 2 | 30 | 22 |
| 4 | 54 | 90 |

These results indicate that the compounds of the present invention possess potent hypoglycemic and hypolipidemic actions. It is clear, therefore, that these compounds are useful as agents for the prophylaxis or treatment of diabetes, hyperlipidemia (especially hypertriglyceridemia), impaired glucose tolerance, and the like.

### Test Example 2

### Total cholesterol lowering action and plasma anti-arteriosclerosis index-increasing action in mice

Test compound was mixed in a powdery diet (CE-2, Japan Clea) at the concentration of 0.005%, and freely given to KKA^{y} mice (12 weeks old, 5 mice per group), a model of obesity and type II diabetes, for four days. During this period, water was given freely. Blood was sampled from orbital venous plexus and plasma was separated and analyzed for components. Total cholesterol levels were determined using L type Wako Cholesterol (Wako Pure Chemical Industries, Ltd.). Precipitation reagent for apoB containing lipoprotein (Wako Pure Chemical Industries, Ltd.) was added to a part of the plasma to allow precipitation of non-HDL lipoprotein, and cholesterol (HDL cholesterol) in the resulting supernatant was determined. The plasma anti-arteriosclerosis index [(HDL cholesterol/total cholesterol)×100] was calculated using these cholesterol levels. The results are given in Table 2.

In the Table, "Total cholesterol lowering action (%)" represents the percent reduction (%) of total cholesterol level in the test compound administration group, when the total cholesterol level in the test compound non-administration group is taken as 100%. "Plasma anti-arteriosclerosis index-increasing action (%)" represents a percent increase (%) of plasma anti-arteriosclerosis index in the test compound administration group, when the plasma anti-arteriosclerosis index in the test compound non-administration group-is taken as 100%.

**Table 2**

| Test compound (Example No.) | Total cholesterol lowering action (%) | Plasma anti-arteriosclerosis index-increasing action (%) |
|---|---|---|
| 4 | 22 | 39 |

These results indicate that the compound of the present invention possesses total cholesterol lowering and plasma anti-arteriosclerosis index-increasing actions. It is clear, therefore, that the compound of the present invention is useful as an agent for the prophylaxis or treatment of arteriosclerosis and the like, by improving plasma lipoprotein profiles of hypercholesterolemia or hypo-HDL-cholesterolemia.

### Test Example 3

### (PPARγ-RXRα heterodimer ligand activity)

A PPARγ:RXRα:4ERPP/CHO-K1 cells obtained in Reference Example 5 to be described later were cultured in HAM F12 medium (produced by NISSUI PHARMACEUTICAL CO., LTD.) containing 10% fetal bovine serum (produced by Life Technologies, Inc., USA) and then inoculated to a 96-well white plate (produced by Corning Costar Corporation, USA) at the density of 2×10⁴ cells/well, and cultured in a CO₂ gas incubator at 37°C overnight.

After washing the 96 well white plate with PBS (phosphate-buffered saline), 90 µl of HAM F12 medium containing 0.1% fatty acid-free bovine serum albumin (BSA) and 10 µl of test compound were added, which was followed by culture in a CO₂ gas incubator at 37°C for 48 hours. After removing the medium, 40 µl of PIKKAGENE 7.5 (produced by Wako Pure Chemical Industries, Ltd.) was added. After stirring, the luciferase activity was determined using Lumistar (produced by BMG Labtechnologies GmbH, Germany).

A fold induction was calculated based on the luciferase activity of each test compound where the luciferase activity in the test compound non-administration group was taken as 1. The values of the test compound concentration and the fold induction were analyzed using PRISM 2.01 (produced by GraphPad Software Inc., USA) to calculate the EC₅₀ values, the effective concentration of the test compound for 50% of the maximum fold induction. The results are shown in Table 3.

**Table 3**

| Test compound (Example No.) | EC₅₀ (µM) |
|---|---|
| 2 | 1.6 |
| 4 | 0.0014 |

As demonstrated, the compounds of the present invention have potent PPARγ-RXRα heterodimer ligand activity.

### Reference Example 1

### (cloning of human PPARγ gene)

A human PPARγ gene was cloned using a heart cDNA (produced by Toyobo Co., Ltd., trade name: QUICK-Clone cDNA) as a template by means of a PCR method employing a primer set shown below which was prepared with reference to the base sequence of PPARγ gene reported by Greene et al (Gene Expr., 1995, Vol. 4 (4-5), page 281 - 299).

PAG-U: 5′-GTG GGT ACC GAA ATG ACC ATG GTT GAC ACA GAG-3′
(SEQ ID Number: 1)

PAG-L: 5′-GGG GTC GAC CAG GAC TCT CTG CTA GTA CAA GTC-3′
(SEQ ID Number: 2)

The PCR reaction was performed by Hot Start method using AmpliWax PCR Gem 100 (produced by TAKARA SHUZO CO., LTD.). First, 2 µl of 10×LA PCR Buffer, 3 µl of 2.5 mM dNTP solution, 2.5 µl each of 12.5 µM primer solutions and 10 µl of sterilized distilled water were mixed to give a lower layer mixture. Human heart cDNA (1 µl, 1 ng/ml) as a template, 3 µl of 10×LA PCR Buffer, 1 µl of 2.5 mM dNTP solution, 0.5 µl of TaKaRa LA Taq DNA polymerase (produced by TAKARA SHUZO CO., LTD.) and 24.5 µl of sterilized distilled water were mixed to give an upper layer mixture.

To the lower layer mixture described above was added one unit of AmpliWax PCR Gem 100 (produced by TAKARA SHUZO CO., LTD.), and the mixture was treated at 70°C for 5 minutes and then in ice for 5 minutes. The upper layer mixture was then added to the mixture to give the reaction mixture of PCR. A tube containing the reaction mixture was set on a thermal cycler (produced by Perkin Elmer, USA) and then treated at 95°C for 2 minutes. After repeating the cycle of 95°C for 15 seconds and 68°C for 2 minutes 35 times, the tube was treated at 72°C for 8 minutes.

The PCR product thus obtained was subjected to electrophoresis on agarose gel (1%), and 1.4 kb DNA fragment containing PPARγ gene was recovered from the gel, and then inserted into pT7 Blue-T vector (produced by TAKARA SHUZO CO., LTD.) to give a plasmid pTBT-hPPARγ.

### Reference Example 2

### (cloning of human RXRα gene)

A human RXRα gene was cloned using a kidney cDNA (produced by Toyobo Co., Ltd., trade name: QUICK-Clone cDNA) as a template by means of a PCR method employing a primer set shown below which was prepared with reference to the base sequence of RXRα gene reported by Mangelsdorf, D. J. et al (Nature, 1990, Vol. 345 (6272), page 224 - 229).

XRA-U: 5′-TTA GAA TTC GAC ATG GAC ACC AAA CAT TTC CTG-3′
(SEQ ID Number: 3)

XRA-L: 5′-CCC CTC GAG CTA AGT CAT TTG GTG CGG CGC CTC-3′
(SEQ ID Number: 4)

The PCR reaction was performed by Hot Start method using AmpliWax PCR Gem 100 (produced by TAKARA SHUZO CO., LTD.). First, 2 µl of 10×LA PCR Buffer, 3 µl of 2.5 mM dNTP solution, 2.5 µl each of 12.5 µM primer solutions and 10 µl of sterilized distilled water were mixed to give a lower layer mixture. Human kidney cDNA (1 µl, 1 ng/ml) as a template, 3 µl of 10×LA PCR Buffer, 1 µl of 2.5 mM dNTP solution, 0.5 µl of TaKaRa LA Taq DNA polymerase (produced by TAKARA SHUZO CO., LTD.) and 24.5 µl of sterilized distilled water were mixed to give an upper layer mixture.

To the lower layer mixture described above was added one unit of AmpliWax PCR Gem 100 (produced by TAKARA SHUZO CO., LTD.), and the mixture was treated at 70°C for 5 minutes and then in ice for 5 minutes. Then, the upper layer mixture was added to the mixture to give the reaction mixture of PCR. A tube containing the reaction mixture was set on a thermal cycler (produced by Perkin Elmer, USA) and treated at 95°C for 2 minutes. After repeating the cycle of 95°C for 15 seconds and 68°C for 2 minutes 35 times, the tube was treated at 72°C for 8 minutes.

The PCR product thus obtained was subjected to electrophoresis on agarose gel (1%), and 1.4 kb DNA fragment containing RXRα gene was recovered from the gel, and then inserted into pT7 Blue-T vector (produced by TAKARA SHUZO CO., LTD.) to give a plasmid pTBT-hRXRa.

### Reference Example 3

### (Construction of plasmids for expressing Human PPARγ, RXRα)

A 7.8 kb FspI-NotI fragment of plasmid pVgRXR (produced by Invitrogen, USA) was ligated to a 0.9 kb FspI-NotI fragment containing RXRα gene of plasmid pTBT-hRXRα obtained in Reference Example 2 to give plasmid pVgRXR2. Then, pVgRXR2 was digested with BstXI and then treated with T4DNA polymerase (produced by TAKARA SHUZO CO., LTD.) to obtain a blunt terminal. Then digestion at KpnI gave a 6.5 kb DNA fragment.

On the other hand, plasmid pTBT-hPPARγ obtained in Reference Example 1 was digested with Sal I and then treated with T4DNA polymerase (produced by TAKARA SHUZO CO., LTD.) to obtain a blunt terminal. Then digestion at KpnI gave a 1.4 kb DNA fragment containing human PPARγ gene.

The both DNA fragments were ligated to construct plasmid pVgRXR2-hPPARγ.

### Reference Example 4

### (Construction of reporter plasmids)

A DNA fragment containing PPAR-responding element (PPRE) of an acyl CoA oxidase was prepared using the following 5'-terminal phosphorylated synthetic DNA.

PPRE-U: 5'-pTCGACAGGGGACCAGGACAAAGGTCACGTTCGGGAG-3' (SEQ
ID Number: 5)

PPRE-L: 5′-pTCGACTCCCGAACGTGACCTTTGTCCTGGTCCCCTG-3′ (SEQ
ID Number: 6)

First, PPRE-U and PPRE-L were annealed and inserted to Sal I site of plasmid pBlue Script SK+. By determining the base sequence of the inserted fragment, plasmid pBSS-PPRE4 in which 4 PPREs were ligated in tandem was selected.

A HSV thymidine kinase minimum promoter (TK promoter) region was cloned using pRL-TK vector (produced by Promega, USA) as a template by means of a PCR method employing a primer set shown below which was prepared with reference to the base sequence of the promoter region of thymidine kinase gene reported by Luckow, B et al (Nucleic Acids Res., 1987, Vol. 15(13), p. 5490)

TK-U: 5'-CCCAGATCTCCCCAGCGTCTTGTCATTG-3' (SEQ ID Number:
7)

TK-L: 5′-TCACCATGGTCAAGCTTTTAAGCGGTC-3′ (SEQ ID Number:
8)

The PCR reaction was performed by Hot Start method using AmpliWax PCR Gem 100 (TAKARA SHUZO CO., LTD.). First, 2 µl of 10×LA PCR Buffer, 3 µl of 2.5 mM dNTP solution, 2.5 µl each of 12.5 µM primer solutions and 10 µl of sterilized distilled water were mixed to give a lower layer mixture. 1 µl of pRL-TK vector (produced by Promega, USA) as a template, 3 µl of 10×LA PCR Buffer, 1 µl of 2.5 mM dNTP solution, 0.5 µl of TaKaRa LA Taq DNA polymerase (produced by TAKARA SHUZO CO., LTD.) and 24.5 µl of sterilized distilled water were mixed to give an upper layer mixture.

To the lower layer mixture described above, added was one unit of AmpliWax PCR Gem 100 (produced by TAKARA SHUZO CO., LTD.), which was treated at 70°C for 5 minutes and then in ice for 5 minutes. Then, the upper layer mixture was added to the mixture to give the reaction mixture of PCR. A tube containing the reaction mixture was set on a thermal cycler (produced by Perkin Elmer, USA) and then treated at 95°C for 2 minutes. After repeating the cycle of 95°C for 15 seconds and 68°C for 2 minutes a further 35 times, the tube was treated at 72°C for 8 minutes.

The PCR product thus obtained was subjected to electrophoresis on agarose gel (1%), and 140 b DNA fragment containing TK promoter was recovered from the gel, and then inserted into pT7 Blue-T vector (produced by TAKARA SHUZO CO., LTD.). By digesting the plasmid thus obtained with the restriction enzymes Bg1 II and NcoI, a fragment containing TK promoter was obtained, which was ligated to the Bg1 II-NcoI fragment of plasmid pGL3-Basic vector (produced by Promega, USA) to give plasmid pGL3-TK.

A 4.9 kb NheI-XhoI fragment of plasmid pGL3-TK thus obtained was ligated to a 200 b NheI-XhoI fragment of plasmid pBSS-PPRE4 to give plasmid pGL3-4ERPP-TK.

This plasmid pGL3-4ERPP-TK thus obtained was digested with BamHI (produced by TAKARA SHUZO CO., LTD.) and then treated with T4DNA polymerase (produced by TAKARA SHUZO CO., LTD.) to form a blunt terminal, whereby giving a DNA fragment.

On the other hand, pGFP-Cl (produced by Toyobo Co., Ltd.) was digested with Bsu36I (NEB) and then treated with T4DNA polymerase (produced by TAKARA SHUZO CO., LTD.) to form a blunt terminal whereby giving a 1.6 kb of a DNA fragment.

The both DNA fragments were ligated to construct a reporter plasmid pGL3-4ERPP-TK neo.

### Reference Example 5

### (Introduction of human PPARγ and RXRα expression plasmid and reporter plasmid into CHO-K1 cell and establishment of expressed cell)

After a CHO-K1 cell cultured in a 750 ml tissue culture flask (produced by Corning Costar Corporation, USA) containing HAM F12 medium (produced by NISSUI PHARMACEUTICAL CO., LTD.) supplemented with 10% Fetal Bovine Serum (produced by Life Technologies, Inc., USA) was scraped by treating with 0.5 g/L trypsin-0.2 g/L EDTA (ethylenediaminetetraacetic acid) (produced by Life Technologies, Inc., USA), the cell was washed with PBS (phosphate-buffered saline) (produced by Life Technologies, Inc., USA), centrifuged (1000 rpm, 5 minutes), and then suspended in PBS. Subsequently, a DNA was introduced into the cell under the conditions shown below using GENE PULSER (produced by Bio-Rad Laboratories, USA).

Namely, to a cuvette having a 0.4 cm gap, added were 8×10⁶ cells and 10 µg of plasmid pVgRXR2-hPPARγ obtained in Reference Example 3 and 10 µg of reporter plasmid pGL3-4ERPP-TK neo obtained in Reference Example 4, which was subjected to electroporation at the voltage of 0.25 kV under the capacitance of 960 µF. Subsequently, the cell was transferred into a HAM F12 medium containing 10% Fetal Bovine Serum and cultured for 24 hours and then the cell was scraped again and centrifuged, and then suspended in HAM F12 medium containing 10% Fetal Bovine Serum supplemented with 500 µg/ml of GENETICIN (produced by Life Technologies, Inc., USA) and 250 µg /ml of ZEOCIN (produced by Invitrogen, USA) and diluted to the density of 10⁴ cells/ml upon inoculation to a 96-well plate (produced by Corning Costar Corporation, USA), which was cultured in a CO₂ gas incubator at 37°C, whereby giving a GENETICIN- and ZEOCIN-resistant transformant.

Subsequently, after the transformant cell line thus obtained was cultured in a 24-well plate (produced by Corning Costar Corporation, USA), selected was a cell line in which the luciferase was expressed and induced, i.e., PPARγ:RXRα:4ERPP/CHO-K1 cell by addition of 10 µM pioglitazone hydrochloride.

### Reference Example 6

To a solution of 6-(5-methyl-2-phenyl-4-oxazolylmethoxy)-2-naphthaldehyde (4.86 g) in methanol (150 ml)-tetrahydrofuran (50 ml) was slowly added sodium borohydride (533 mg) at 0°C. The mixture was stirred at room temperature for 1 hour and water was added to the reaction mixture. The precipitated 6-(5-methyl-2-phenyl-4-oxazolylmethoxy)-2-naphthalene methanol as crystals (4.69 g, yield 96%) was collected by filtration. To a mixture of the crystals (4.60 g) and tetrahydrofuran (100 ml) was added thionyl chloride (1.58 g) and the mixture was stirred at room temperature for 1 hour. Ice water was added to the reaction mixture and the mixture was extracted with ethyl acetate. The ethyl acetate layer was washed with water, dried (MgSO₄) and concentrated to give 4-(6-chloromethyl-2-naphthoxymethyl)-5-methyl-2-phenyloxazole as crystals (4.11 g, yield 85%).
NMR (CDCl₃) δ: 2.47 (3H, s), 4.74(2H, s), 5.12(2H, s), 7.4-7.55(4H, m), 7.7-7.8(3H, m), 8.0-8.1 (2H, m).

### Reference Example 7

To a solution of 6-(5-methyl-2-phenyl-4-oxazolylmethoxy)pyridine-3-carbaldehyde (13.0 g) in tetrahydrofuran (150 ml)-methanol (10 ml) was slowly added sodium borohydride (835 mg) at 0°C. The mixture was stirred for 30 minutes and water was added to the reaction mixture. The mixture was extracted with ethyl acetate and the ethyl acetate layer was washed with water, dried (MgSO₄) and concentrated to give 6-(5-methyl-2-phenyl-4-oxazolylmethoxy)pyridine-3-methanol as crystals. The crystals were recrystallized from acetone-isopropyl ether to give colorless prism crystals (12.4 g, yield 95%), melting point: 121-122°C.

### Reference Example 8

To a mixture of 6-(5-methyl-2-phenyl-4-oxazolylmethoxy)pyridine-3-methanol (12.2 g) and toluene (200 ml) was added thionyl chloride (5.39 g) and the mixture was stirred at room temperature for 1 hour. Ice water was added to the reaction mixture and the mixture was neutralized with saturated aqueous sodium hydrogen carbonate solution and extracted with ethyl acetate. The ethyl acetate layer was washed with water, dried (MgSO₄) and concentrated. The residue was subjected to silica gel column chromatography. From the fraction eluted with ethyl acetate-hexane (1:3, v/v), 5-chloromethyl-2-(5-methyl-2-phenyl-4-oxazolylmethoxy)pyridine was obtained as crystals (11.7 g, yield 90%). The crystals were recrystallized from ethyl acetate-hexane to give colorless prism crystals, melting point: 86-87°C.

### Example 1

Sodium hydride (60%, oil, 200 mg) was gradually added to a solution of 4-(6-chloromethyl-2-naphthoxymethyl)-5-methyl-2-phenyloxazole (1.82 g) and methyl (E)-4-hydroxyimino-4-phenylbutyrate (1.10 g) in N,N-dimethylformamide (20 ml) and the mixture was stirred at room temperature for 1 hour. The reaction mixture was poured into 1N hydrochloric acid with cooling and the mixture was extracted with ethyl acetate. The ethyl acetate layer was washed with water, dried (MgSO₄) and concentrated. The residue was subjected to silica gel column chromatography. From the fraction eluted with ethyl acetate-hexane (1:4, v/v), methyl (E)-4-[6-(5-methyl-2-phenyl-4-oxazolylmethoxy)-2-naphthylmethoxyimino]-4-phenylbutyrate was obtained as crystals (1.80 g, yield 67%). The crystals were recrystallized from ethyl acetate-isopropyl ether to give pale-yellow prism crystals, melting point: 111-113°C.

### Example 2

Methyl (E)-4-[6-(5-methyl-2-phenyl-4-oxazolylmethoxy)-2-naphthylmethoxyimino]-4-phenylbutyrate (1.60 g) was dissolved in ethanol (10 ml) and 1N aqueous sodium hydroxide solution (5 ml) was added. The mixture was stirred at room temperature for 1 hour. 1N Hydrochloric acid was added to the reaction mixture and the precipitated crystals were collected by filtration. The crystals were recrystallized from ethanolisopropyl ether to give (E)-4-[6-(5-methyl-2-phenyl-4-oxazolylmethoxy)-2-naphthylmethoxyimino]-4-phenylbutyric acid as colorless prism crystals (1.25 g, yield 80%), melting point: 135-136°C.

### Example 3

Sodium hydride (60%, oil, 102 mg) was gradually added to a solution of 5-chloromethyl-2-(5-methyl-2-phenyl-4-oxazolylmethoxy)pyridine (800 mg) and methyl (E)-4-hydroxyimino-4-phenylbutyrate (527 mg) in N,N-dimethylformamide (5 ml) under a nitrogen atmosphere at 0°C and the mixture was stirred at room temperature for 1.5 hours. Diethyl ether (300 ml) was added to the reaction mixture and the mixture was washed with water, dried (MgSO₄) and concentrated. The residue was subjected to silica gel column chromatography. From the fraction eluted with ethyl acetate-hexane (1:3, v/v), methyl (E)-4-[6-(5-methyl-2-phenyl-4-oxazolylmethoxy)-3-pyridylmethoxyimino]-4-phenylbutyrate (1.07 g, yield 87%) was obtained as a colorless oil.
NMR (CDCl₃) δ: 2.48(3H, s), 2.49-2.58(2H, m), 3.0-3.1(2H, m), 3.63(3H, s), 5.15(2H, s), 5.31(2H, s), 6.82(2H, d, J=8.4Hz), 7.34-7.46(6H, m), 7.57-7.69(3H, m), 7.97-8.05(2H, m), 8.22(1H, d, J=2.2Hz).

### Example 4

Methyl (E)-4-[6-(5-methyl-2-phenyl-4-oxazolylmethoxy)-3-pyridylmethoxyimino]-4-phenylbutyrate (1.00 g) was dissolved in a mixture of tetrahydrofuran (10 ml), water (6 ml) and methanol (6 ml) and lithium hydroxide monohydrate (259 mg) was added. The mixture was stirred at room temperature for 2 hours. 1N Hydrochloric acid (6.2 ml) was added to the reaction mixture and the mixture was extracted with ethyl acetate. The ethyl acetate layer was washed with water, dried (MgSO₄) and concentrated. The residue was recrystallized from ethyl acetate-hexane to give (E)-4-[6-(5-methyl-2-phenyl-4-oxazolylmethoxy)-3-pyridylmethoxyimino]-4-phenylbutyric acid as colorless prism crystals (843 mg, yield 87%), melting point: 106-107°C.

| **Formulation Example 1** (production of capsule) | |
|---|---|
| 1) compound of Example 4 | 30 mg |
| 2) microcrystalline cellulose | 10 mg |
| 3) lactose | 19 mg |
| 4) magnesium stearate | 1 mg |
| total | 60 mg |
| 1), 2), 3) and 4) are mixed and filled in gelatin capsules. | |

| **Formulation Example 2** (production of tablet) | |
|---|---|
| 1) compound of Example 4 | 30 g |
| 2) lactose | 50 g |
| 3) corn starch | 15 g |
| 4) calcium carboxymethylcellulose | 44 g |
| 5) magnesium stearate | 1 g |
| total of 1000 tablets | 140 g |

The entire amounts of 1), 2) and 3), and 30 g of 4) are kneaded with water, dried in vacuo and milled. The milled powders are mixed with 14 g of 4) and 1 g of 5) and the mixture is tableted with a tableting machine, whereby 1000 tablets containing 30 mg of compound of Example 4 per tablet are obtained.

### Industrial Applicability

The compound of the present invention and the pharmaceutical composition of the present invention have low toxicity and can be used as, for example, an agent for the prophylaxis or treatment of diabetes (e.g., type I diabetes, type II diabetes, gestational diabetes etc.); an agent for the prophylaxis or treatment of hyperlipidemia (e.g., hypertriglyceridemia, hypercholesterolemia, hypo-high-density-lipoproteinemia, postprandial hyperlipidemia etc.); an agent for enhancing insulin sensitivity; an agent for improving insulin resistance; an agent for the prophylaxis or treatment of impaired glucose tolerance (IGT); and an agent for preventing progress from impaired glucose tolerance to diabetes.

The compound of the present invention and the pharmaceutical composition of the present invention can be also used as, for example, an agent for the prophylaxis or treatment of diabetic complications (e.g., neuropathy, nephropathy, retinopathy, cataract, macroangiopathy, osteopenia, hyperosmolar diabetic coma, infectious diseases (e.g., respiratory infection, urinary tract infection, gastrointestinal infection, dermal soft tissue infections, inferior limb infection and the like), diabetic gangrene, xerostomia, hypocusis, cerebrovascular disorder, peripheral blood circulation disorder, etc.), obesity, osteoporosis, cachexias (e.g., cancerous cachexia, tuberculous cachexia, diabetic cachexia, hemopathic cachexia, endocrinopathic cachexia, infectious cachexia, cachexia due to acquired immunodeficiency syndrome), fatty liver, hypertension, polycystic ovary syndrome, renal diseases (e.g., diabetic nephropathy, glomerular nephritis, glomerulosclerosis, nephrotic syndrome, hypertensive nephrosclerosis, terminal renal disease and the like), muscular dystrophy, myocardial infarction, angina pectoris, cerebrovascular disorders (e.g., cerebral infarction, cerebral apoplexy), insulin resistance syndrome, syndrome X, hyperinsulinemia, hyperinsulinemia-induced sensory disorder, tumors (e.g., leukemia, breast cancer, prostate cancer, skin cancer and the like), irritable bowel syndrome, acute or chronic diarrhea and visceral obesity syndrome.

Also, the compound of the present invention and the pharmaceutical composition of the present invention can be used for ameliorating the condition of bellyache, nausea, vomiting, dysphoria in epigastrium and the like, each of which is accompanied by peptic ulcer, acute or chronic gastritis, biliary dyskinesia, cholecystitis and the like.

Further, the compound of the present invention and the pharmaceutical composition of the present invention can control (enhance or inhibit) appetite, and therefore, can be used as an agent for treating, for example, leanness and cibophobia (the weight increase in administration subjects suffering from leanness or cibophobia) or an agent for treating obesity.

Of the compounds of the present invention, compound (I) can be used as an agent for the prophylaxis or treatment of inflammatory diseases (e.g., rheumatoid arthritis, spondylitis deformans, osteoarthritis, lumbago, gout, postoperative or traumatic inflammation, remission of swelling, neuralgia, laryngopharyngitis, cystitis, hepatitis (including non-alcoholic steatohepatitis), pneumonia, pancreatitis, inflammatory bowel disease, ulcerative colitis and the like), arteriosclerosis (e.g., atherosclerosis and the like), and the like.

## Claims

1. A compound represented by the formula 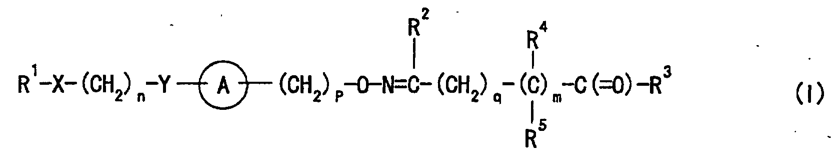 wherein
R¹ is an optionally substituted hydrocarbon group or an optionally substituted heterocyclic group;
X is a bond, an oxygen atom, a sulfur atom, or a group represented by -CO-, -CS-, -CR⁶(OR⁷)- or -NR⁸- (R⁶ and R⁸ are each a hydrogen atom or an optionally substituted hydrocarbon group, and R⁷ is a hydrogen atom or a hydroxy-protecting group);
n is an integer of 0 to 3;
Y is an oxygen atom, a sulfur atom, or a group represented by -SO-, -SO₂-, -NR⁸-, -CONR⁸- or -NR⁸CO- (R⁸ is a hydrogen atom or an optionally substituted hydrocarbon group);
ring A is a heterocycle optionally having 1 to 3 additional substituents or a hydrocarbon ring optionally having 1 to 3 additional substituents;
p is an integer of 1 to 8;
R² is a hydrogen atom, an optionally substituted hydrocarbon group or an optionally substituted heterocyclic group;
q is an integer of 0 to 6;
m is 0 or 1;
R³ is a group represented by -OR⁹ (R⁹ is a hydrogen atom or an optionally substituted hydrocarbon group) or -NR¹⁰R¹³ (R¹⁰ and R¹¹ are the same or different and each is a hydrogen atom, an optionally substituted hydrocarbon group, an optionally substituted heterocyclic group, or an optionally substituted acyl group, and R¹⁰ and R¹¹ may be taken together to form a ring) ;
R⁴ and R⁵ are the same or different and each is a hydrogen atom or an optionally substituted hydrocarbon group, and R⁴ and R² may be taken together to form a ring, provided that, when ring A is an optionally substituted indole, Y is not an oxygen atom or a sulfur atom; when Y is an oxygen atom, a sulfur atom, -SO-, -SO₂- or -NR⁸- (R⁸ is as defined above), ring A is not a benzene ring optionally having substituents; and when Y is an oxygen atom and ring A is an optionally substituted 4-pyrone, an optionally substituted 4-pyridone or an optionally substituted pyridine-N-oxide, R² is not a thiazole substituted by an optionally protected amino group or a thiadiazole substituted by an optionally protected amino group,
or a salt thereof.

2. The compound of claim 1, wherein R¹ is an optionally substituted heterocyclic group or an optionally substituted cyclic hydrocarbon group.

3. The compound of claim 1, wherein R¹ is an optionally substituted heterocyclic group.

4. The compound of claim 1, wherein X is a bond.

5. The compound of claim 1, wherein n is 1 or 2.

6. The compound of claim 1, wherein Y is an oxygen atom.

7. The compound of claim 1, wherein the ring A is a pyridine ring optionally having 1 to 3 additional substituents or a naphthalene ring optionally having 1 to 3 additional substituents.

8. The compound of claim 1, wherein p is an integer of 1 to 3.

9. The compound of claim 1, wherein R² is an optionally substituted hydrocarbon group.

10. The compound of claim 1, wherein q is an integer of 0 to 4.

11. The compound of claim 1, wherein R³ is a group represented by -OR⁹ (R⁹ is a hydrogen atom or an optionally substituted hydrocarbon group).

12. The compound of claim 1, which is methyl (E)-4-[6-(5-methyl-2-phenyl-4-oxazolylmethoxy)-2-naphthylmethoxyimino]-4-phenylbutyrate;
(E)-4-[6-(5-methyl-2-phenyl-4-oxazolylmethoxy)-2-naphthylmethoxyimino]-4-phenylbutyric acid;
methyl (E)-4-[6-(5-methyl-2-phenyl-4-oxazolylmethoxy)-3-pyridylmethoxyimino]-4-phenylbutyrate; or
(E)-4-[6-(5-methyl-2-phenyl-4-oxazolylmethoxy)-3-pyridylmethoxyimino]-4-phenylbutyric acid.

13. A prodrug of the compound of claim 1.

14. A pharmaceutical composition comprising a compound represented by the formula 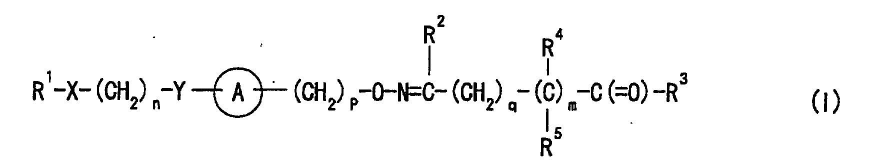 wherein each symbol is as defined in claim 1, a salt thereof or a prodrug thereof.

15. An agent for the prophylaxis or treatment of diabetes, which comprises a compound represented by the formula 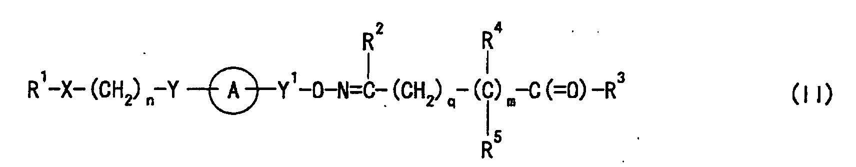 wherein
R¹ is an optionally substituted hydrocarbon group or an optionally substituted heterocyclic group;
X is a bond, an oxygen atom, a sulfur atom, or a group represented by -CO-, -CS-, -CR⁶(OR⁷)- or -NR⁸- (R⁶ and R⁸ are each a hydrogen atom or an optionally substituted hydrocarbon group, and R⁷ is a hydrogen atom or a hydroxy-protecting group);
n is an integer of 0 to 3;
Y is an oxygen atom, a sulfur atom, or a group represented by -SO-, -SO₂-, -NR⁸-, -CONR⁸- or -NR⁸CO- (R⁸ is a hydrogen atom or an optionally substituted hydrocarbon group);
ring A is a heterocycle optionally having 1 to 3 additional substituents or a hydrocarbon ring optionally having 1 to 3 additional substituents;
Y¹ is an optionally substituted divalent hydrocarbon residue having 1 to 8 carbon atoms in a main chain;
R² is a hydrogen atom, an optionally substituted hydrocarbon group or an optionally substituted heterocyclic group;
q is an integer of 0 to 6;
m is 0 or 1;
R³ is a group represented by -OR⁹ (R⁹ is a hydrogen atom or an optionally substituted hydrocarbon group) or -NR¹⁰R¹¹ (R¹⁰ and R¹¹ are the same or different and each is a hydrogen atom, an optionally substituted hydrocarbon group, an optionally substituted heterocyclic group, or an optionally substituted acyl group, and R¹⁰ and R¹¹ may be taken together to form a ring);
R⁴ and R⁵ are the same or different and each is a hydrogen atom or an optionally substituted hydrocarbon group, and R⁴ and R² may be taken together to form a ring, provided that, when Y is an oxygen atom, a sulfur atom, -SO-, -SO₂- or -NR⁸- (R⁸ is as defined above), ring A is not a benzene ring optionally having substituents;
or a salt thereof or a prodrug thereof.

16. An agent for the prophylaxis or treatment of hyperlipidemia, which comprises a compound represented by the formula 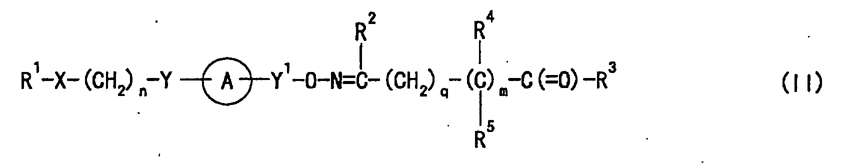 wherein each symbol is as defined in claim 15, a salt thereof or a prodrug thereof.

17. An agent for the prophylaxis or treatment of impaired glucose tolerance, which comprises a compound represented by the formula 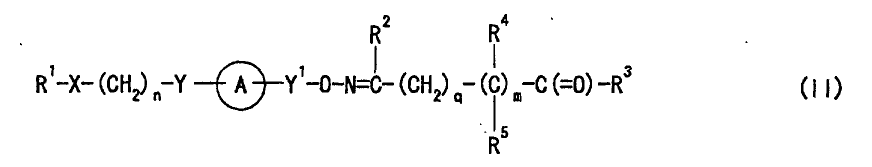 wherein each symbol is as defined in claim 15, a salt thereof or a prodrug thereof.

18. An agent for regulating a retinoid-related receptor function, which comprises a compound represented by the formula 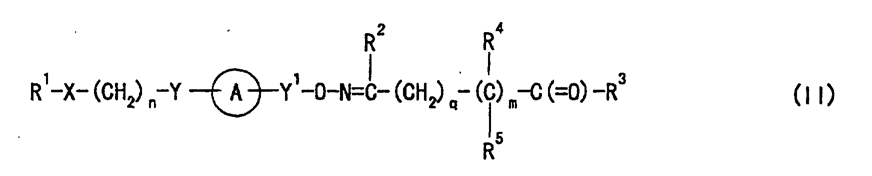 wherein each symbol is as defined in claim 15, a salt thereof or a prodrug thereof.

19. The agent of claim 18, which is a ligand for a peroxisome proliferator-activated receptor.

20. The agent of claim 18, which is a retinoid X receptor ligand.

21. An agent for improving insulin resistance, which comprises a compound represented by the formula 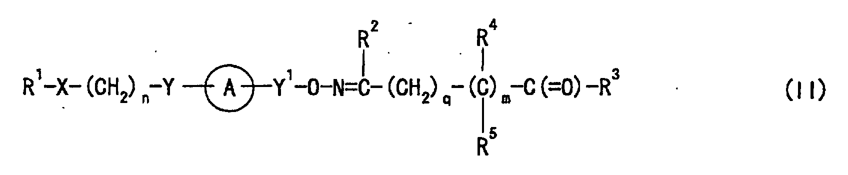 wherein each symbol is as defined in claim 15, a salt thereof or a prodrug thereof.

22. A method for the prophylaxis or treatment of diabetes, which comprises administering an effective amount of a compound represented by the formula 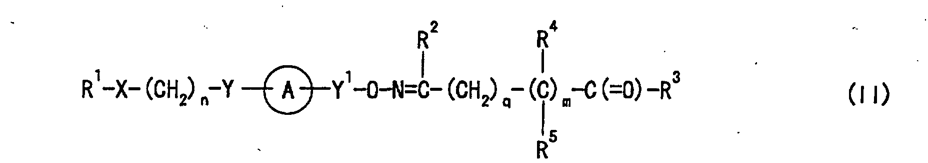 wherein each symbol is as defined in claim 15, a salt thereof or a prodrug thereof to a mammal.

23. A method for the prophylaxis or treatment of hyperlipidemia, which comprises administering an effective amount of a compound represented by the formula 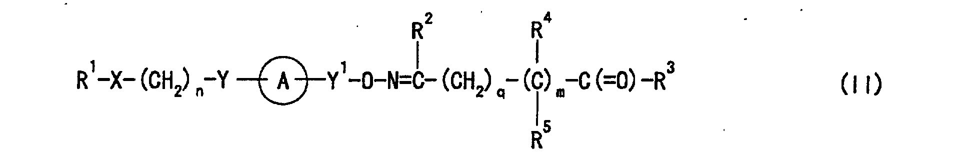 wherein each symbol is as defined in claim 15, a salt thereof or a prodrug thereof to a mammal.

24. A method for the prophylaxis or treatment of impaired glucose tolerance, which comprises administering an effective amount of a compound represented by the formula 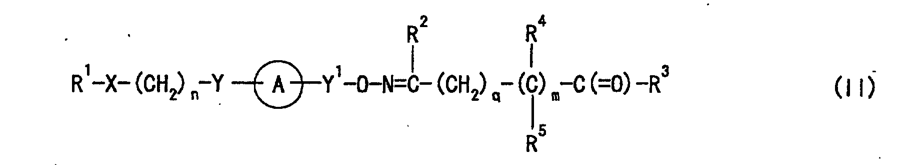 wherein each symbol is as defined in claim 15, a salt thereof or a prodrug thereof to a mammal.

25. A method for regulating a retinoid-related receptor function, which comprises administering an effective amount of a compound represented by the formula 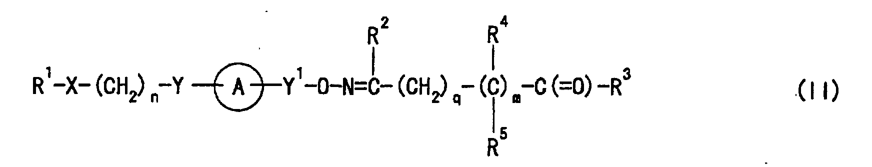 wherein each symbol is as defined in claim 15, a salt thereof or a prodrug thereof to a mammal.

26. Use of a compound represented by the formula 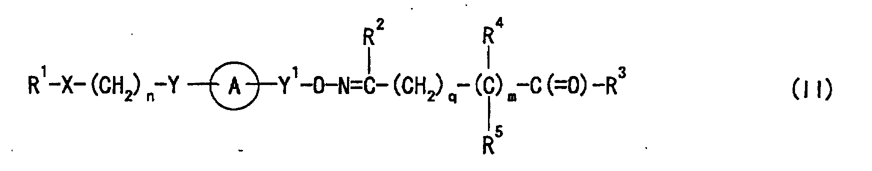 wherein each symbol is as defined in claim 15, a salt thereof or a prodrug thereof for the production of an agent for the prophylaxis or treatment of diabetes.

27. Use of a compound represented by the formula 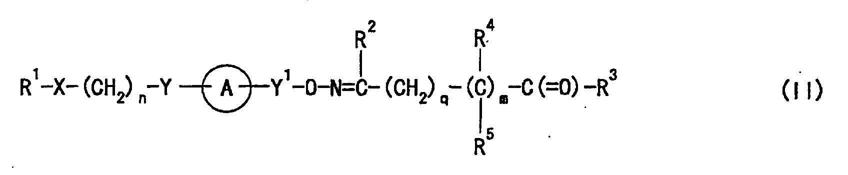 wherein each symbol is as defined in claim 15, a salt thereof or a prodrug thereof for the production of an agent for the prophylaxis or treatment of hyperlipidemia.

28. Use of a compound represented by the formula 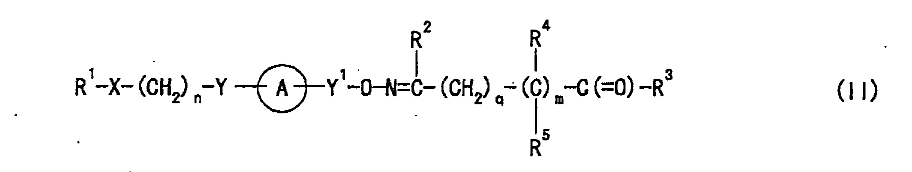 wherein each symbol is as defined in claim 15, a salt thereof or a prodrug thereof for the production of an agent for the prophylaxis or treatment of impaired glucose tolerance.

29. Use of a compound represented by the formula 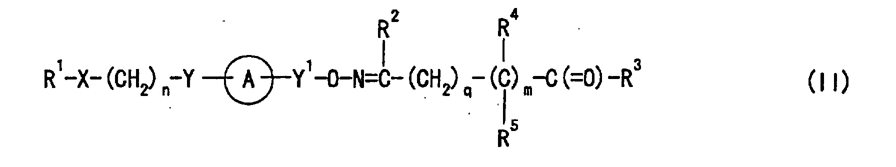 wherein each symbol is as defined in claim 15, a salt thereof or a prodrug thereof for the production of an agent for regulating a retinoid-related receptor function.
